# EUROPÄISCHE PATENTSCHRIFT

(11) **EP 1 863 817 B1**
(45) Veröffentlichungstag und Bekanntmachung des Hinweises auf die Patenterteilung: **28.05.2008**
(21) Anmeldenummer: 06723254.6
(22) Anmeldetag: 07.03.2006
(51) Int. Cl.: C07D 487/04, A61P 3/00, A61P 9/00, A61K 31/4184

(54) **SUBSTITUIERTE, BIZYKLISCHE 8-PYRROLIDINO-BENZIMIDAZOLE, VERFAHREN ZU IHRER HERSTELLUNG UND IHRE VERWENDUNG ALS ARZNEIMITTEL**
SUBSTITUTED, BICYCLIC 8-PYRROLIDINO-BENZIMIDAZOLES, METHOD FOR THEIR PRODUCTION AND THEIR USE AS MEDICAMENTS
8-PYRROLIDINO-BENZIMIDAZOLES BICYCLIQUES SUBSTITUES, LEUR PROCEDE DE PRODUCTION, ET LEUR UTILISATION EN TANT QUE MEDICAMENTS

(30) Priorität: 19.03.2005 DE 102005012872
(43) Veröffentlichungstag der Anmeldung: 12.12.2007
(73) Patentinhaber: sanofi-aventis, 75013 Paris (FR)
(72) Erfinder: DEFOSSA, Elisabeth, 65510 Idstein (DE); SCHOENAFINGER, Karl, 63755 Alzenau (DE); JAEHNE, Gerhard, 65929 Frankfurt (DE); BUNNING, Christian, 65926 Frankfurt am Main (DE); TSCHANK, Georg, 55270 Essenheim (DE); WERNER, Ulrich, 56357 Miehlen (DE)
(74) Vertreter: Fischer, Hans-Jürgen
(86) Internationale Anmeldenummer: PCT/EP2006/002055
(87) Internationale Veröffentlichungsnummer: WO 2006/099940

(56) Entgegenhaltungen:
- US-A1- 2002 107 396
- US-A1- 2003 008 861

## Beschreibung

Die Erfindung betrifft substituierte, bizyklische 8-Pyrrolidino-benzimidazole sowie deren physiologisch verträgliche Salze und physiologisch funktionelle Derivate.

Es sind bereits strukturähnliche Verbindungen im Stand der Technik beschrieben (siehe WO 20040 72069). US-A-2002/0107396 beschreibt Benzimidazol-Derivate als Medikamente zur Behandlung von Diabetes Typ 2.

Der Erfindung lag die Aufgabe zugrunde, Verbindungen zur Verfügung zu stellen, die eine therapeutisch verwertbare, Blutzucker senkende Wirkung entfalten.

Die Erfindung betrifft daher Verbindungen der Formel I, 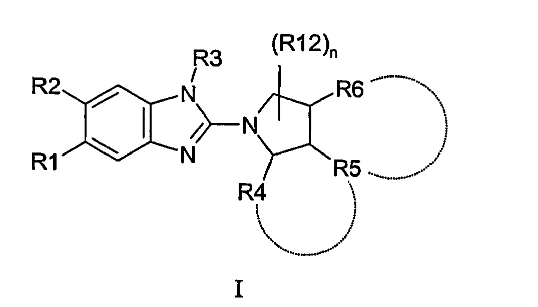 worin bedeuten
- R1, R2: unabhängig voneinander H, CONR20R21, NR22COR23 oder NR24R25, wobei nicht beide Reste R1 oder R2 Wasserstoff entsprechen können;
- R20, R21: unabhängig voneinander H, (C₁-Cₗ₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Alkylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
R22, R23 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Akyl, S-(C₁-C₆)-Alkyl, S(O)-(C₁-C₆)-Alkyl oder S(O)₂-(C₁-C₆)-Alkyl;
R24, R25 unabhängig voneinander H, (C₁-C₁₀)-Alkyl, (C₃-C₁₀)-Cycloalkyl, (C₂-C₁₀)-Alkenyl, (₂-C₁₀)-Alkinyl, (C₆-C₁₀)-Aryl, Heterocyclyl, (C₁-C₆)-Alkylen-(C₆-C₁₀)-Aryl, (C₁-C₆)-Allcylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Akyl, S(O)-(C₁-C₆)-Alkyl oder s(O)₂-(C₁-C₆)-Alkyl;
R3 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;
wobei die Bedeutung unsubstituiertes oder substituiertes Piperidin-4-yl für R3 ausgenommen ist;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Akylen-(C₆-C₁₀)-Aryl oder (C₁-C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR11 ersetzt ist, wobei R6 gleich H oder R12 ist, oder
R5 und R6 bilden zusammen eine 3 bis 5 gliedrige Alkylenkette, worin eine CH₂-Gruppe durch NR11 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alklyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl, (C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl, (C₃-C₈)-Cycloalkyl, NH₂, NH(C₁-C₆)-Akyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
n 0, 1 oder 2; wobei Verbindungen ausgenommen sind in denen die Reste gleichzeitig bedeuten
R1 oder R2 gleich CONR20M1 und R3 gleich Wasserstoff oder CH₃;
sowie deren physiologisch verträglichen Salze.

Bevorzugt sind Verbindungen der Formel I, worin ein oder mehrere Reste die folgende Bedeutung haben:
- R1, R2: unabhängig voneinander H, CONR20R21, NR22COR23 oder NR24R25, wobei nicht beide Reste R1 oder R2 Wasserstoff entsprechen können;
- R20, R21: unabhängig voneinander H, (C₁-C₁₀)-Alkyl, (C₃-C₁₀)-Cycloakyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Alkylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
- R22, R23: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)2-(C₁-C₆)Alkyl;
- R24, R25: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
- R3: (C₂-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Allcylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;
wobei die Bedeutung unsubstituiertes oder substituiertes Piperidin-4-yl für R3 ausgenommen ist;
- R7, R8: unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Allcylen-SR9, (C₁-C₆)-Allcylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Alkylen-Heterocyclyl;
- R9, R10: unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Allcylen-Heterocyclyl;
- R4 und R5: bilden zusammen eine 3-5-gliedrige Alkylenkette, worin eine CH₂-Gruppe durch NR1ersetzt ist, wobei R6 gleich H oder R12 ist, oder
- R5 und R6: bilden zusammen eine 3 bis 5 gliedrige Alkylenkette, worin eine CH₂-Gruppe durch NR11 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- R11: H, (C₁-C₆)-Alkyl, (C₃-C₈)-Cycloalkyl, (C₁-C₄)-Alkylen-Aryl oder (C₁-C₄)-Alkylen-Heterocyclyl;
- R12: F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- n: 0, 1 oder 2;
sowie deren physiologisch verträglichen Salze.

Besonders bevorzugt sind Verbindungen der Formel I, worin ein oder mehrere Reste die folgende Bedeutung haben:
- R1, R2: unabhängig voneinander H, CONR20R21, NR22COR23 oder NR24R25, wobei nicht beide Reste R1 oder R2 Wasserstoff entsprechen können;
- R20, R21: unabhängig voneinander H, (C₁-C₁₀)-Alkyl, (C₃-C₁₀)-Cycloallcyl, (C₂-C₁₀)-Allcenyl, (C₂-C₁₀)-Allcinyl, (C₆-C₁₀)-Aryl, Heterocyclyl, (C₁-C₆)-Allcylen-(C₆-C₁₀)-Aryl oder (C₁-C₆)-Alkylen-(C₆-Cₗo)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Akyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
- R22, R23: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
- R24, R25: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyayl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
- R3: (C₂-C₁₀)-Alkyl, (C₃-C₁₀)-Cycloalkyl, (C₂-C₁₀)-Alkenyl, (C₂-C₁0)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Allcylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C6)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;*
wobei die Bedeutung unsubstituiertes oder substituiertes Piperidin-4-yl für R3 ausgenommen ist;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Allcylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Allcylen-Heterocyclyl;
R9, R10 unabhängig voneinander H, (C₁-C₆)-Alky, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR1 ersetzt ist, wobei R6 gleich H oder R12 ist, oder
R5 und R6 bilden zusammen eine 3 bis 5 gliedrige Alkylenkette, worin eine CH₂-Gruppe durch NR11 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-CYcloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl, (C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl₎ (C₃-C₈)-Cyacloalkyl, NH₂, NH(C₁-C₆)-Alkyl, NH(C₃-C₇)-Cycloalkyl, N((C₁-C₆)-Allkyl)₂ oder O-(C₁-C₆)-Alkyl, wobei die Alkylgruppen ein- oder mehrfach mit F, Cl, Br oder I substituiert sein können;
n 0, 1 oder 2; sowie deren physiologisch verträglichen Salze.

Ganz besonders bevorzugt sind Verbindungen der Formel I, worin ein oder mehrere Reste die folgende Bedeutung haben:
- R1, R2: unabhängig voneinander H, Benzylaminocarbonyl, Benzoylamino, wobei die Benzylaminocarbonyl- und Benzoylaminoreste ein- oder mehrfach substituiert sein können mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N(C₁-C₆)-Alkyl)₂, SO₂-CH₃, COOH, COO-(C₁-C₆)-Alkyl, CONH₂;
wobei nicht beide Reste R1 oder R2 Wasserstoff entsprechen können;
- R3: (C₂-C₁₀)-Alkenyl, Benzyl, wobei oder Benzylrest ein- oder mehrfach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl N((C₁-C₆)-Alkyl)₂, SO₂-CH₃, COOH, COO-(C₁-C₆)-Alkyl, CONH₂;
- R4 und R5: bilden zusammen eine 3-5-gliedrige Alkylenkette, worin eine CH₂-Gruppe durch NH ersetzt ist, wobei die Alkylgruppen ein- oder mehrfach mit F, Cl, Br oder I substituiert sein können;
- R11: H, (C₁-C₆)-Alkyl, (C₃-C₈)-Cycloalkyl, (C₁-C₄)-Alkylen-Aryl oder (C₁-C₄)-Alkylen-Heterocyclyl;
- n: 0;
sowie deren physiologisch verträglichen Salze.

Die Erfindung bezieht sich weiterhin auf die Verwendung der Verbindungen der Formel I, 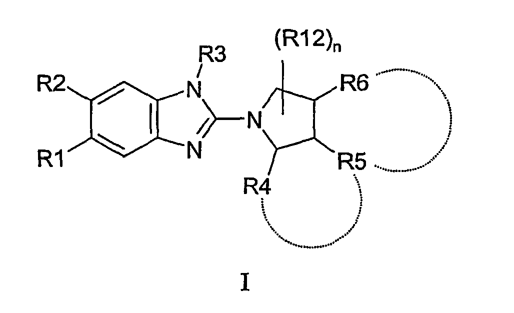 worin bedeuten
- R1, R2: unabhängig voneinander H, CONR20R21, NR22C0R23 oder NR24R25;
- R20, R21: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Akylen-(C₆-C₁₀)-Heterocyayl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
- R22, R23: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁C₆)-Alkyl;
- R24, R25: unabhängig voneinander H, (C₁-C₁₀)-Alkyl, (C₃-C₁₀-Cycloalkyl, (C₂-C₁₀)-Alkenyl, (C₂-C₁₀)-Alkinyl, (C₆-C₁₀)-Aryl, Heterocyclyl, (C₁-C₆)-Allcylen-(C₆-C₁₀)-Aryl, (C₁-C₆)-Akylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Akyl, O-(C₁-C₆)-Alkyl, S-(C₁-C₆)-Alkyl, S(O)-(C₁-C₆)-Alkyl oder S(O)₂-(C₁-C₆)-Alkyl;
- R3: unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Allcenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(0)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Allcylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;
- R7, R8: unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Allcylen-SR9, (C₁-C₆)-Alkylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Allcylen-Heterocyclyl;
- R9, R10: unabhängig voneinander H, (C₁-C₆)-Allcyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
- R4 und R5: bilden zusammen eine 3-5-gliedrige Alkylenkette, worin eine CH₂-Gruppe durch NR11 ersetzt ist, wobei R6 gleich H oder R12 ist, oder
- R5 und R6: bilden zusammen eine 3 bis 5 gliedrige Alkylenkette, worin eine CH₂-Gruppe durch NR11 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- R11: H, (C₁-C₆)-Alkyl, (C₃-C₈)-Cycloalkyl, (C₁-C₄)-Alkylen-Aryl oder (C₁-C₄)-Alkylen-Heterocyclyl;
- R12: F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
- n: 0, 1 oder 2;
sowie derer physiologisch verträglichen Salze zur Herstellung eines Medikaments zur Blutzuckersenkung.

Die Erfindung bezieht sich auf Verbindungen der Formel I, in Form ihrer Razemate, razemischen Mischungen und reinen Enantiomeren sowie auf ihre Diastereomeren und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff , Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Allcyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ Heterocyclus, S0₂-(C₁-C₆)- Alkyl, SO₂-(CH₂)ₙ-Aryl, SO₂-(CH₂)ₙ Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Allcyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Allcyl)-Aryl, N((C₁-C₆)-Alkyl)-CO-N((C₁-C₆)-Alkyl)-Heterocyclus, N((C₁-C₆)-Allcyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Allcyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl, 2-Methyl-but-2-en-4-yl.

Die Alkenylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Akyl)(CH₂)ₙ-Heterocyclus, SO₂-N((CH₂)ₙ-Aryl)₂, S₂-N(CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Allcyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Butinyl, Hexinyl.

Die Alkinylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-C0-(C₁-C₆)-Heterocyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂),-Aryl, S-(CH₂)ₙ-Heterocyclus, SO-(C₁-C₆)-Alkyl, SO-(CH₂),-Aryl, SO-(CH₂)ₙ Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Allcyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Akyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)ₙ-Aryl und O-(CH₂)ₙ Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Alkyl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Alkyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heteroeyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(ArY1)₂, Aryl, O-(CH₂)ₙ-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)n-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Allcyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Allcyl)-CO-Aryl, N((C₁-C₆)-Alkyl)-CO-Heterocyclus, N((C₁-C₆)-Alkyl)-COO-Aryl, N((C₁-C₆)-Alkyl)-COO-Heterocyclus, N((C₁-C₆)-Alkyl)-CO-NH-(C₁-C₆)-Akyl), N((C₁-C₆)-Alkyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁-C₆)-Alkyl)-Aryl, N((C₁-C₆)-Alkyl)-CO-N((C₁-C₆)-Alkyl)-Heterocyclus, N ((C₁-C₆)- Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Allcyl)-Aryl, N(Heterocyclus)-CO-N((C₁-C₆)-Alkyl)-Awl, N(Aryl)-CO-N-(Aryl)₂, N(Heterocyclus)-CO-N-(Aryl)₂, Aryl, O-(CH₂)ₙ-Aryl und O-(CH₂)ₙ Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂.

Unter Heterocyclyl, Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit Benzolkernen kondensiert ist. Der Heterocyclus bzw. der heterocyclische Rest kann aromatisch, gesättigt aliphatisch oder teilweise ungesättigt aliphatisch sein.

Geeignete Heterocyclyl-Reste, bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-Pyridyl.

Umfasst sind weiterhin ein oder mehrfach benzoannelierte Derivate dieser Heterocyclen.

Die Heterocyclischen Ringe bzw. Heterocyclische Reste können ein oder mehrfach mit geeigneten Gruppen substituiert sein, wie z.B: F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, Cycloalkyl, (C₂-C₆)-Alkenyl, (C₂-C₆)-Alkinyl, O-(C₁-C₆)-Alkyl, O-CO-(C₁-C₆)-Alkyl, O-CO-(C₁-C₆)-Aryl, O-CO-(C₁-C₆)-Heterocyclus; PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂(CH₂)ₙ-Heterocyclus, SO₂-NH(CH₂)ₙ-Aryl, SO₂-NH(CH₂)ₙ-Heterocyclus, SO₂-N((C₁-C₆)-Alkyl)(CH₂)ₙ-Aryl, SO₂-N((C₁-C₆)-Alkyl)(CH₂)ₙ-Heterocyclus, SO₂-N((CH₂)ₙ-Aryl)₂, SO₂-N((CH₂)ₙ-Heterocyclus)₂ wobei n = 0 - 6 sein kann und der Arylrest oder Heterocyclische Rest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; C(=NH)(NH₂), NH₂, NH-(C₁-C₆)-Alkyl, N((C₁-C₆)-Alkyl)₂, NH(C₁-C₇)-Acyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, N((C₁-C₆)-Alkyl)-CO-(C₁-C₆)-Alkyl, N((C₁-C₆)-Alkyl)-COO-(C₁-C₆)-Alkyl, N((C₁-C₆)-Alkyl)-CO-Aryl, N((C₁-C₆)-Alkyl)-CO-Heterocyclus, N((C₁-C₆)-Alkyl)-COO-Aryl, N((C₁-C₆)- Alkyl)-COO- Heterocyclus, N((C₁-C₆)-Alkyl)-CO-NH-(C₁-C₆)-Alkyl), N((C₁-C₆)-Allcyl)-CO-NH-Aryl, N((C₁-C₆)-Alkyl)-CO-NH-Heterocyclus, N((C₁-C₆)-Alkyl)-CO-N((C₁-C₆)-Alkyl)₂, N((C₁-C₆)-Alkyl)-CO-N((C₁ - C₆)-Alkyl)-Aryl, N((C₁-C₆)-Alkyl)-CO-N((C₁-C₆)-Alkyl)-Heterocyclus, N((C₁-C₆)-Alkyl)-CO-N-(Aryl)₂, N((C₁-C₆)-Alkyl)-CO-N-(Heterocyclus)₂, N(Aryl)-CO-(C₁-C₆)-Alkyl, N(Heterocyclus)-CO-(C₁-C₆)-Alkyl, N(Aryl)-COO-(C₁-C₆)-Alkyl, N(Heterocyclus)-COO-(C₁-C₆)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-(C₁-C₆)-Alkyl), N(Heterocyclus)-CO-NH-(C₁-C₆)-Alkyl), N(Aryl)-CO-NH-Aryl, N(Heterocyclus)-CO-NH-Aryl, N(Aryl)-CO-N((C₁-C₆)-Alkyl)₂, N(Heterocyclus)-CO-N((C₁-C₆)-Alkyl)₂, N(Aryl)-CO-N((C₁-C₆)-Alkyl)-Aryl, N(Heterocyclus)-CO-N((C₁-C₆)-Alkyl)-Aryl, N(Aryl)-CO-N-(Aryl)₂, N(Heterocyclus)-CO-N-(Aryl)₂, Aryl, O-(CH₂)"-Aryl und O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂ SO₂-CH₃, COOH, COO-(C₁-C₆)-Alkyl oder CONH₂.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 ng pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2005, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin oder Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP 2004/00269, PCT/EP 2003/05815, PCT/EP 2003/05814, PCT/EP 2003/05816, EP 0114531 oder US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501 oder Gl 262570, verabreicht.

Bei einer Ausführungsform der Erfindung werden die Verbindungen der Formel I in Kombination mit PPAR alpha Agonist, wie z.B. GW 9578 oder GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/LTS 11490 oder DE10142734.4 beschrieben verabreicht.

Bei einer Ausführungsform der Erfindung werden die Verbindungen der Formel I in Kombination mit einem Fibrat, wie z.B. Fenofibrat, Clofibrat oder Bezafibrat, verabreicht.

Bei einer Ausführungsform der Erfindung werden die Verbindungen der Formel I in Kombination mit einem MTP-Inhibitor, wie z.B. Implitapide , BMS-201038 oder R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin oder Colesevelam, verabreicht.

Bei einer Ausführungsform der Erfindung werden die Verbindungen der Formel I in Kombination mit einem LDL-Rezeptorinducer (siehe US 6,342,512), wie z.B. HIVIR1171 oder HMR1586, verabreicht.

Bei einer Ausfühungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausfiihrungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) Antagonisten, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid, verabreicht. Bei einer Ausführungsform werden die Verbindungen der Formel I in Kombination mit einem Biguanid, wie z.B. Metformin, verabreicht.

Bei einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Adenosin Al Agonisten, wie z. B. solchen, die in EP 0912520 oder PCT/EP06749 beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc., verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure- {4-[(4-amino-quinazolin-2-ylamino)-methyl]-cyclohexylimethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid, (WO O1/91752)), Orexin-Antagonisten (z.B. 1-(2-Methyl-benzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)), TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451)), CB1 (Cannabinoid Rezeptor 1) Rezeptor Antagonisten (z.B. Rimonabant oder die in WO 02/28346 genannten Wirkstoffe), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl)-essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (siehe z.B. WO 00/71549), 5HT-Agonisten z.B. 1-(3-Ethyl-benzofuran-7-yl)-piperazin Oxalsäuresalz (WO 01/09111)), Bombesin-Agonisten, Galanin-Antagonisten, Wachstumshormon (z.B. humanes Wachstumshormon), Wachstumshormon freisetzende Verbindungen (6-Benzyloxy-1-(2-düsopropylamino-ethylcarbamoyl)-3,4-dihydro-1H-isochinolin-2-carbonsäuretertiärbutylester (WO 01/85695)), TRH-Agonisten (siehe z.B. EP 0 462 884), entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (z.B. Bromocriptin oder Doprexin), Lipase/Amylase-Inhibitoren (siehe z.B. WO 00/40569), PPAR-Modulatoren (siehe z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin.
Bei einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

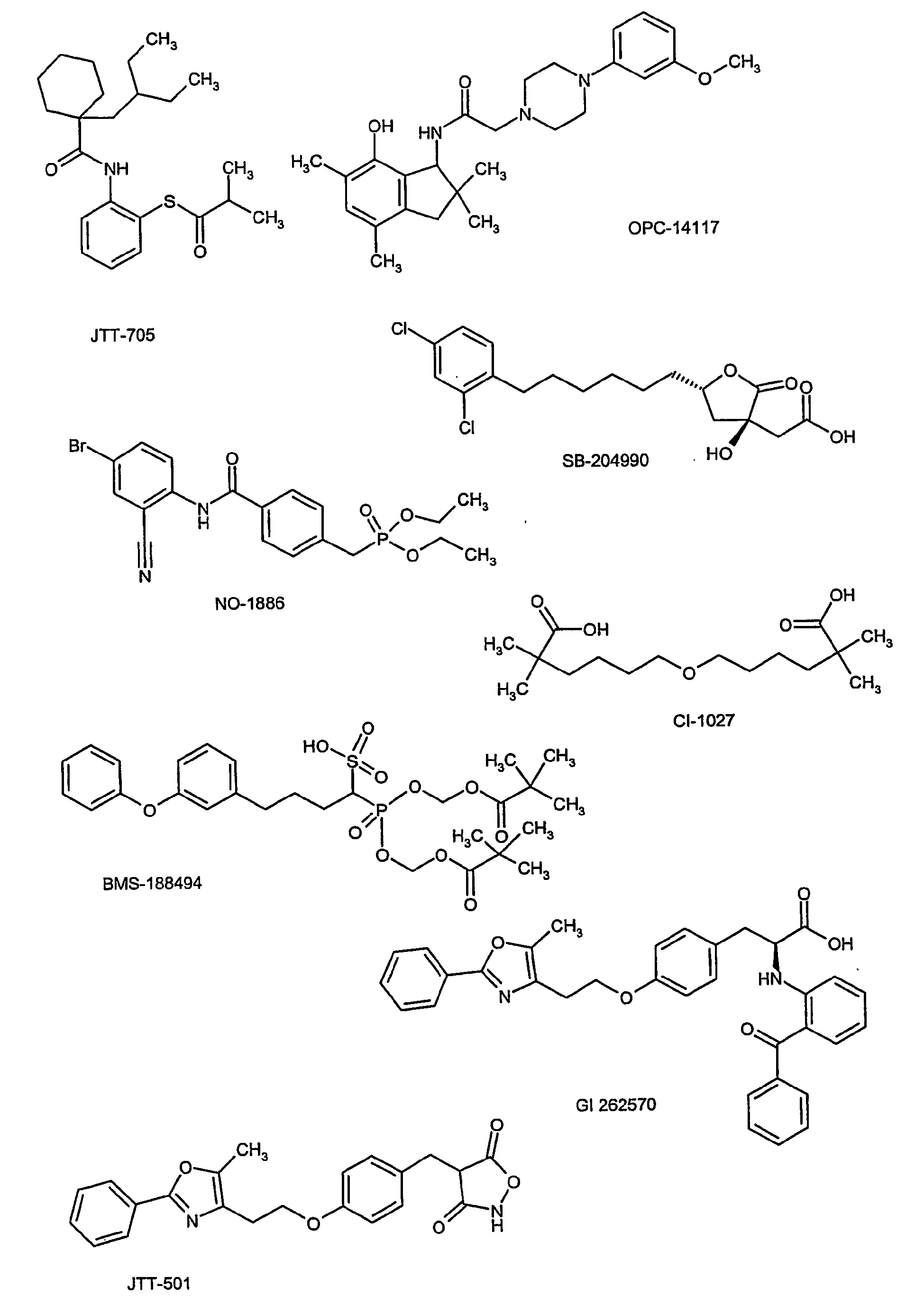

Die Verbindungen der Formel I lassen sich dadurch herstellen, dass man geeignete Ausgangsstoffe der Formel II, worin X eine Austrittsgruppe, wie Chlor, Brom, Jod, Sulfonyloxi, Sulfinyl oder Sulfoxyl, bedeutet, mit einer Verbindung der Formel IV ggf. in Gegenwart von geeigneten Basen und in geeigneten Lösemitteln umsetzt.

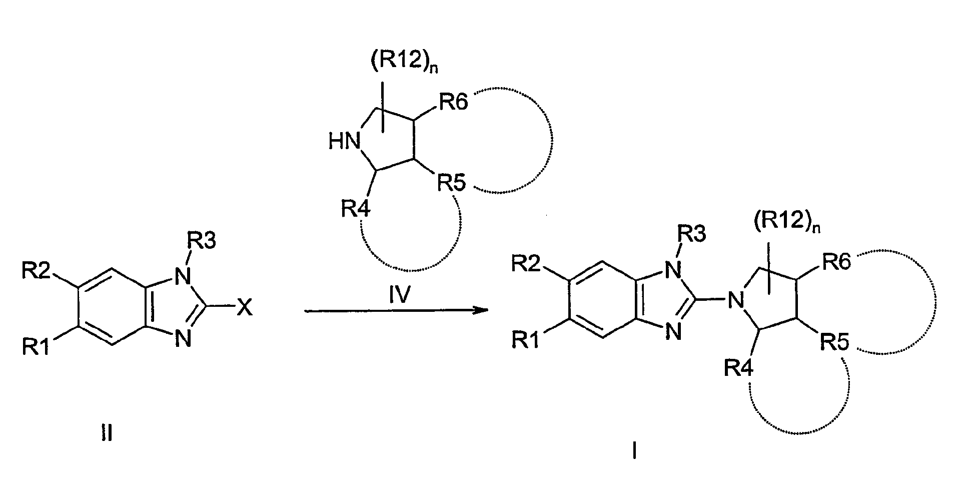

In den Fällen, wo R11 Wasserstoff bedeutet, kann es zweckmäßig sein, den Rest IV in an der Stickstofffunktion geschützter Form einzusetzen und die Schutzgruppe nach erfolgter Reaktion mit II wieder abzuspalten. Solche geeignete Schutzgruppen und die Verfahren der Einführung und Abspaltung sind bekannt (Siehe:Theodora W. Greene and Peter G. M. Wuts, Protective Groups in Organic Synthesis, 3rd Edition, John Wiley & Sons, Inc., New York, 1999) Die Halogenverbindungen der Formel II können nach bekannten Verfahren wie beispielsweise durch Halogenierung der entsprechenden H-, Hydroxy- oder Thio-Verbindung (Formel II, X = H, OH oder SH) erhalten werden. Geeignete Halogenierungsmittel können beispielhaft Halogene, wie Chlor und Brom, N-Bromsuccinimid, Phosphorpentachlorid oder Phosphoroxychlorid sein.
Die Synthese von Verbindungen der Formel II ist in der Literatur beschrieben. Sie können beispielsweise durch Kondensation von substituierten Diaminobenzolderivaten mit Aldehyden in Gegenwart eines Oxidationsmitteln (z.B. Luftsauerstoff, Sauerstoff, Iod, Oxone, Chinonen, Peroxiden, usw.) oder alternativ mit Carbonsäuren, Nitrilen oder Amiden ohne oder in Gegenwart eines Katalysators hergestellt werden.
Die Synthese der bizyklischen Amine IV kann nach literaturbekannten Verfahren erfolgen.

So ist die Herstellung verschiedener Derivate dieser Strukturklasse, wie zum Beispiel von Octahydro-pyrrolo(3,4-b)-pyrrol und 1-Methyl-octahydro-pyrrolo[3,4-b]pyrrol, in EP 0 393 424 beschrieben worden.
Einige Derivate der Formel IV, wie zum Beispiel Octahydro-pyrrolo[3,4-b]pyridin oder Octahydro-pyrrolo[3,4-c]pyridin sind kommerziell erhältlich.

Die nachfolgend aufgeführten tabellarischen Beispiele dienen zur Erläuterung der Erfindung, ohne diese jedoch einzuschränken.

**Tabelle 1:**

| 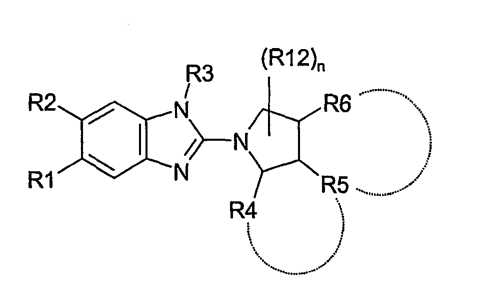 | | | | | | | |
|---|---|---|---|---|---|---|---|
| Bsp. | R1 | R2 | R3 | R4-R5 | R5-R6 | R12 | Salz |
| 1 | H | Benzoylamino | CH₂-CH=C(CH₃)₂ | -CH₂-NH-CH₂- | R6=H | H | TFA |
| 2 | H | Benzoylamino | CH₂-CH=C(CH₃)₂ | R4=H | -CH₂-NH-CH₂- | H | TFA |
| 3 | H | Benzylaminocarbonyl | Benzyl | -CH₂-NH-CH₂- | R6 = H | H | TFA |
| 4 | Benzylaminocarbonyl | H | Benzyl | -CH₂-NH-CH₂- | R6 = H | H | TFA |
| 5 | H | Benzylaminocarbonyl | Benzyl | R4 = H | -CH₂-NH-CH₂- | H | TFA |
| 6 | Benzylaminocarbonyl | | Benzyl | R4=H | -CH₂-NH-CH₂- | H | TFA |

Die Verbindungen der Formel I zeichnen sich durch günstige Wirkungen auf den Lipid- und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-N (Dipeptidyl Peptidase IV) Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen, und zur Behandlung von Drogenmissbrauch.
Weiterhin eignen sie sich zur Behandlung von Krebs, Arthritis, Osteoarthritis, Osteoporose, Schlafstörungen, Schlafapnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:

Messung der DPP-IV Aktivität:

Material:
DPP-IV aus Schweineniere (Sigma, München)
H-Ala-Pro-AFC (Bachem, Weil am Rhein)

Testbedingungen:
DPP-IV (1 mU/ml, Endkonzentration)
H-Ala-Pro-AFC (15µM Endkonzentration)
in Tris/HCl (40 mM, pH 7.4), Gesamtvolumen 0,2 ml

Die Reaktion wurde bei Raumtemperatur für unterschiedliche Zeiträume (typischerweise 10 Minuten) durchgeführt und am Ende der Reaktion durch Zugabe von 20 µl ZnCl₂ (1 M) gestoppt. Der Umsatz von H-Ala-Pro-AFCwurde fluorimetrisch durch Messung der Emission bei 535 nm nach Anregung bei 405 nm bestimmt. Im Falle der Zugabe von Inhibitoren wurde das zugegebene Puffervolumen so angepasst, dass ein Gesamtvolumen der Testmischung von 200 µl eingehalten wurde.
%Hemmung bei einer festen Konzentration wurden wie folgt errechnet:
(1-Enzymaktivität gehemmte Reaktion / Enzymaktivitätungehemmte Reaktion) X 100

**Tabelle 2: Biologische Aktivität von Ausführungsbeispielen:**

| Beispiel | % Hemmung bei 30 µM |
|---|---|
| 1 | 25 |

**Tabelle 3: Biologische Aktivität von Ausführungsbeispielen:**

| Beispiel | % Hemmung bei 10 µM |
|---|---|
| 5 | 87 |
| 6 | 96 |

Aus der Tabelle ist abzulesen, dass die Verbindungen der Formel I die Aktivität der DPP-IV (Dipeptidyl Peptidase IV) hemmen und dadurch zur Senkung des Blutzuckerspiegels geeignet sind.

Nachfolgend wird die Herstellung der Ausführungsbeispiele detailliert beschrieben:

### Beispiel 1

### N-[2-(Hexahydro-pyrrolo[3,4-b]pyrrol-2-yl)-1-(3-methyl-but-2-enyl)-1H-benzimidazol-6-yl]-benzamid

a) 2-Brom-6-nitro-1H-benzimidazol 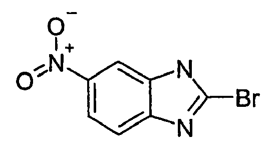
   Eine Suspension von 5,0 g (25,61 mmol) 6-Nitro-1H-benzimidazole-2-thiol in 30 ml Methanol und 10 ml Bromwasserstoff (48%ig in Wasser) wurde auf 5-10°C abgekühlt und mit 5,7 ml (111 mmol) Brom versetzt. Anschließend wurde 1,5 Stunden bei 5-10°C gerührt und auf Eiswasser gegossen. Der rote Niederschlag wurde abgesaugt, mit Wasser gewaschen und bei 40°C im Vakuum getrocknet. Man erhielt 4,82 g (78%) des gewünschten Produktes.
b) 2-Brom-1-(3-methyl-but-2-enyl)-5/6-nitro-1H-benzimidazol 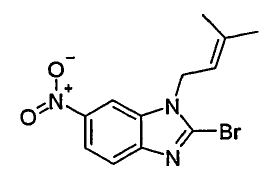
   1,0 g (4,13 mmol) 2-Brom-5/6-nitro-1H-benzimidazol wurden in 20 ml Dimethylformamid gelöst, mit 2,02 g (6,20 mmol) Cäsiumkarbonat versetzt und 30 Minuten bei Raumtemperatur gerührt. 0,677 g (4,54 mmol) 1-Brom-3-methyl-2-buten wurden zugegeben und das Reaktionsgemisch wurde 18 Stunden bei Raumtemperatur gerührt. Der Niederschlag wurde abgesaugt und mit Dimethylformamid gewaschen. Das Filtrat wurde im Vakuum eingeengt und der Rückstand zwischen Dichlormethan und Wasser verteilt. Die organische Phase wurde getrocknet und im Vakuum eingeengt. Das Rohgemisch wurde an Kieselgel getrennt (Laufmittel: Heptan/Essigsäureethylester, Gradient: 90/10 auf 0/100). Man erhielt 766 mg (60%) des gewünschten Produktes.
   MS: m/z = 311 (M+H)⁺.
c) 1-[1-(3-Methyl-but-2-enyl)-6-nitro-1H-benzimidazol-2-yl]-hexahydro-pyrrolo[3,4-b]pyrrol-5-carbonsäure-tert-butylester 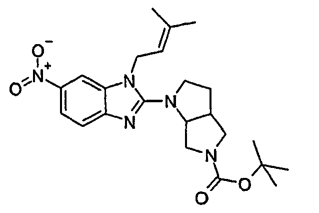
   81 mg (0,38 mmol) Hexahydro-pyrrolo[3,4-b]pyrrol-5-carbonsäure-tert-butylester wurden in 1 ml Dimethylformamid gelöst und mit 170 mg (0,52 mmol) Cäsiumkarbonat versetzt und 30 Minuten bei Raumtemperatur gerührt. 108 mg (0,35 mmol) 2-Brom-1-(3-methyl-but-2-enyl)-5/6-nitro-1H-benzimidazol wurden in 5 ml Dimethylformamid gelöst und langsam zugegeben. Das Reaktionsgemisch wurde 9 Stunden bei 90°C gerührt und anschließend zwischen Dichlormethan und Wasser verteilt. Die organische Phase wurde getrocknet und im Vakuum eingeengt. Das Rohgemisch wurde an Kieselgel getrennt (Laufmittel:
   Heptan/Essigsäureethylester, Gradient: 90/10 auf 0/100). Man erhielt 44 mg (29%) 1-[1-(3-Methyl-but-2-enyl)-5-nitro-1H-benzimidazol-2-yl]-hexahydro-pyrrolo[3,4-b]pyrrol-S-carbonsäure-tert-butylester und 49 mg (32%) des gewünschten Produktes.
   MS: m/z = 442 (M+H)⁺.
d) 1-[6-Amino-1-(3-methyl-but-2-enyl)-1H-benzimidazol-2-yl]-hexahydro-pyrrolo[3,4-b]pyrrol-5-carbonsäure-tert-butylester 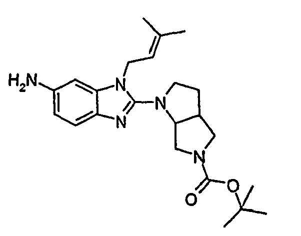
   Zu einer Suspension von 62 mg (1,11 mmol) Eisen und 11 mg (0,20 mmol) Ammoniumchlorid in 0,75 ml Wasser wurde eine Lösung von 49 mg (0,11 mmol) 1-[1-(3-Methyl-but-2-enyl)-6-nitro-1H-benzimidazol-2-yl] -hexahydro-pyrrolo [3,4-b]pyrrol-5 -carbonsäure-tert-butylester in 7,5 ml Ethanol getropft und 3 Stunden bei Rückfluss gekocht. Der Katalysator wurde abfiltriert und mit Ethanol gewaschen. Das Filtrat wurde im Vakuum eingeengt. Die so erhaltenen 43 mg des gewünschten Produktes wurden ohne weitere Reinigung in der nächsten Stufe eingesetzt.
e) 1-[6-Benzoylamino-1-(3-methyl-but-2-enyl)-1H-benzimidazol-2-yl]-hexahydro-pyrrolo[3,4-b]pyrrol-5-carbonsäure-tert-butylester 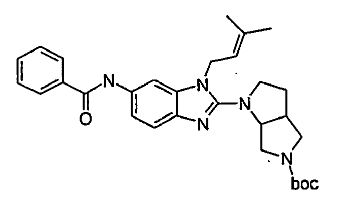
   Zu einer Lösung von 43 mg (0,1 mmol) 1-[6-Amino-1-(3-methyl-but-2-enyl)-1H-benzimidazol-2-yl]-hexahydro-pyrrolo[3,4-b]pyrrol-5-carbonsäure-tert-butylester in 5 ml Dimethylformamid gab man 17 mg (0,05 mmol) Cäsiumkarbonat und rührte 30 Minuten bei Raumtemperatur. Anschließend wurden 12 µl (0,10 mmol) Benzoylchlorid zugegeben und 20 Stunden bei Raumtemperatur gerührt. Das Reaktionsgemisch wurde im Vakuum eingeengt und mittels präparativer HPLC (Acetonitril/Wasser + 0.5% Trifluoressigsäure, Gradient: 20/80 auf 100/0) gereinigt. Man erhielt 10 mg (20%) der gewünschten Verbindung.
f) N-[2-(Hexahydro-pyrrolo[3,4-b]pyrrol-2-yl)-1-(3-methyl-but-2-enyl)-3H-benzimidazol-6-yl]-benzamid 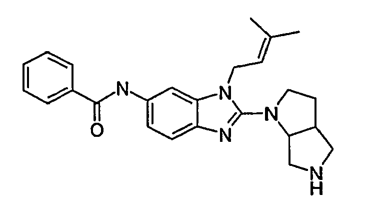
   10 mg (0,02 mmol) 1-[6-Benzoylamino-1-(3-methyl-but-2-enyl)-1H-benzimidazol-2-yl]-hexahydro-pyrrolo[3,4-b]pyrrol-5-carbonsäure-tert-butylester wurden in 200 µl Trifluoressigsäure und 200 µl Wasser gelöst und 19 Stunden bei Raumtemperatur gerührt. Das Reaktionsgemisch wurde mit Wasser verdünnt und gefriergetrocknet. Man erhielt 10 mg (95%) des gewünschten Produktes.
   MS: m/z = 416 (M+H)⁺.

### Beispiel 2

### N-[2-(Hexahydro-pyrrolo[3,4-c]pyrrol-1-yl)-1-(3-methyl-but-2-enyl)-1H-benzimidazol-6-yl]-benzamid (A003454047A)

a) 5-[1-(3-Methyl-but-2-enyl)-6-nitro-1H-benzimidazol-2-yl]-hexahydro-pyrrolo[3,4-c]pyrrol-2-carbonsäure-tert-butylester 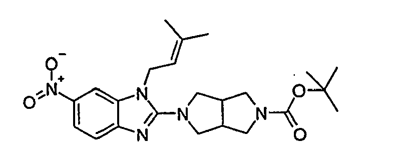
   111 mg (0,52 mmol) Hexahydro-pyrrolo[3,4-c]pyrrol-2-carbonsäure-tert-butylester wurden in 1 ml Dimethylformamid gelöst und mit 170 mg (0,52 mmol) Cäsiumkarbonat versetzt und 30 Minuten bei Raumtemperatur gerührt. 108 mg (0,35 mmol) 2-Brom-1-(3-methyl-but-2-enyl)-5/6-nitro-1H-benzimidazol (Beispiel 1b) wurden in 5 ml Dimethylformamid gelöst und langsam zugegeben. Das Reaktionsgemisch wurde 1,5 Stunden bei 150°C in der Mikrowelle gerührt und anschließend im Vakuum eingeengt. Das Rohgemisch wurde zwischen Dichlormethan und Wasser verteilt. Die organische Phase wurde getrocknet und im Vakuum eingeengt. Das Rohgemisch wurde an Kieselgel getrennt (Laufmittel: Heptan/Essigsäureethylester, Gradient: 90/10 auf 0/100). Man erhielt 74 mg einer Mischfraktion vom gewünschten Produkt mit 5-[1-(3-Methyl-but-2-enyl)-5-nitro-1H-benzimidazol-2-yl]-hexahydro-pyrrolo[3,4-c]pyrrol-2-carbonsäure-tert-butylester und 44 mg (29%) des gewünschten Produktes.
   MS: m/z = 442 (M+H)⁺.
b) 5-[6-Amino-1-(3-methyl-but-2-enyl)-1H-benzimidazol-2-yl]-hexahydro-pyrrolo[3,4-c]pyrrol-2-carbonsäure-tert-butylester 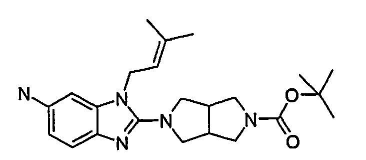
   Zu einer Suspension von 56 mg (1,00 mmol) Eisen und 10 mg (0,18 mmol) Ammoniumchlorid in 0,75 ml Wasser wurde eine Lösung von 44 mg (0,10 mmol) 5-[1-(3-Methyl-but-2-enyl)-6-nitro-1H-benzimidazol-2-yl]-hexahydro-pyrrolo[3,4-c]pyrrol-2-carbonsäure-tert-butylester in 7,5 ml Ethanol getropft und 3 Stunden bei Rückfluss gekocht. Der Katalysator wurde abfiltriert und mit Ethanol gewaschen. Das Filtrat wurde im Vakuum eingeengt. Die so erhaltenen 35 mg des gewünschten Produktes wurden ohne weitere Reinigung in der nächsten Stufe eingesetzt.
c) 5-[6-Benzoylamino-1-(3-methyl-but-2-enyl)-1H-benzimidazol-2-yl]-hexahydro-pyrrolo[3,4-c]pyrrol-2-carbonsäure-tert-butylester 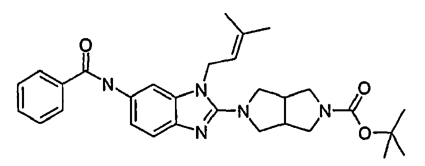
   Zu einer Lösung von 35 mg (0,09 mmol) 5-[6-Amino-1-(3-methyl-but-2-enyl)-1H-benzimidazol-2-yl]-hexahydro-pyrrolo[3,4-c]pyrrol-2-carbonsäure-tert-butylester in 5 ml Dimethylformamid gab man 14 mg (0,04 mmol) Cäsiumkarbonat und rührte 30 Minuten bei Raumtemperatur. Anschließend wurden 10 µl (0,08 mmol) Benzoylchlorid zugegeben und 20 Stunden bei Raumtemperatur gerührt. Das Reaktionsgemisch wurde im Vakuum eingeengt und mittels präparativer HPLC (Acetonitril/Wasser + 0.5% Trifluoressigsäure, Gradient: 20/80 auf 100/0) gereinigt. Man erhielt 10 mg (22%) der gewünschten Verbindung.
d) N-[2-(Hexahydro-pyrrolo[3,4-c]pyrrol-1-yl)-1-(3-methyl-but-2-enyl)-1H-benzimidazol-6-yl]-benzamide 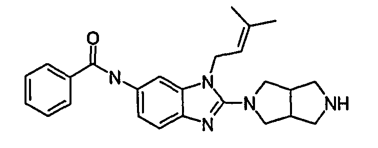
   10 mg (0,02 mmol) 5-[6-Benzoylamino-1-(3-methyl-but-2-enyl)-1H-benzimidazol-2-yl]-hexahydro-pyrrolo[3,4-c]pyrrol-2-carbonsäure-tert-butylester wurden in 200 µl Trifluoressigsäure und 200 µl Wasser gelöst und 19 Stunden bei Raumtemperatur gerührt. Das Reaktionsgemisch wurde mit Wasser verdünnt und gefriergetrocknet. Man erhielt 10 mg (95%) des gewünschten Produktes.
   MS: m/z = 416 (M+H)⁺.

## Patentansprüche

1. Verbindungen der Formel I, 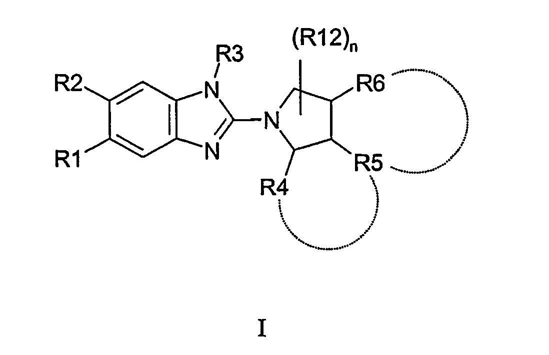 worin bedeuten
R1, R2 unabhängig voneinander H, CONR20R21, NR22COR23 oder NR24R25, wobei nicht beide Reste R1 oder R2 Wasserstoff entsprechen können;
R20, R21 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Alkylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
R22, R23 unabhängig voneinander H, (C₁-C₁₀)-Alkyl, (C₃-C₁₀)-Cycloallcyl, (C₂-C₁₀-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Akylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R24, R25 unabhängig voneinander H, (C₁-C₁₀)-Alkyl, (C₃-C₁₀)-Cycloalkyl, (C₂-C₁₀)-Alkenyl, (C₂-C₁₀)-Akinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Akyl;
R3 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloakyl, (C₁-C₆)-Alkylen-(C₆-C₁₀)-Aryl, (C₁-C₆)- Alkylen-Heterocyclyl, (C₃-C₁₀)-Cycloalkyl, (C₆-C₁₀)-Aryl oder Heterocyclyl;
wobei die Bedeutung unsubstituiertes oder substituiertes Piperidin-4-yl für R3 ausgenommen ist;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR1 ersetzt ist, wobei R6 gleich H oder R12 ist, oder
R5 und R6 bilden zusammen eine 3 bis 5 gliedrige Alkylenkette, worin eine CH₂-Gruppe durch NR1 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl, (C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Allyl, wobei die Alkylgruppen ein- oder mehrfach mit F, Cl, Br oder I substituiert sein können;
n 0, 1 oder 2;
wobei Verbindungen ausgenommen sind in denen die Reste gleichzeitig bedeuten
R1 oder R2 gleich CONR2OR21 und R3 gleich Wasserstoff oder CH₃;
sowie deren physiologisch verträglichen Salze.

2. Verbindungen der Formel I, gemäß Anspruch 1, **dadurch gekennzeichnet, dass** darin bedeuten
R1, R2 unabhängig voneinander H, CONR20R21, NR22COR23 oder NR24R25, wobei nicht beide Reste R1 oder R2 Wasserstoff entsprechen können;
R20, R21 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Alkylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
R22, R23 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyayl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R24, R25 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heteroeyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R3 (C₂-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR⁷)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(O)₂R7, (C₁-C₆)-Alhylen-SR7, (C₁-C₆)-Allcylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cyaoakyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;
wobei die Bedeutung unsubstituiertes oder substituiertes Piperidin-4-yl für R3 ausgenommen ist;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR11 ersetzt ist, wobei R6 gleich H oder R12 ist, oder
R5 und R6 bilden zusammen eine 3 bis 5 gliedrige Alkylenkette, worin eine CH₂-Gruppe durch NR1 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl, (C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Akyl)₂ oder O-(C₁-C₆)-Alkyl, wobei die Alkylgruppen ein- oder mehrfach mit F, Cl, Br oder I substituiert sein können;
n 0, 1 oder 2;
sowie deren physiologisch verträglichen Salze.

3. Verbindungen der Formel I, gemäß Anspruch 1 oder 2, **dadurch gekennzeichnet, dass** darin bedeuten
R1, R2 unabhängig voneinander H, CONR20R21, NR22C0R23 oder NR24R25, wobei nicht beide Reste R1 oder R2 Wasserstoff entsprechen können;
R20, R21 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Alkylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
wobei die Bedeutung Phenyl, anneliert mit stickstoffhaltigen 5-Ring-Heteroaromaten, für R20 und R21 ausgenommen ist;
R22, R23 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R24, R25 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R3 (C₂-C₁₀)-Alkyl, (C₃-C₁₀)-Cycloalkyl, (C₂-C₁₀)-Alkenyl, (C₂-C₁₀)-Alkinyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Akinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloalkyl, (C₁-C₆)-Alkylen-(C₆-C₁₀)- Aryl, (C₁-C₆)-Alkylen-Heterocyclyl, (C₃-C₁₀)-Cycloalkyl, (C₆-C₁0)-Aryl oder Heterocyclyl;
wobei die Bedeutung unsubstituiertes oder substituiertes Piperidin-4-yl für R3 ausgenommen ist;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆)-Alkylen-OR9, (C₁-C₆)-Alkylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Alkylen-(C₆-C₁₀)-Aryl oder (C₁-C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR1 ersetzt ist, wobei R6 gleich H oder R12 ist, oder
R5 und R6 bilden zusammen eine 3 bis 5 gliedrige Alkylenkette, worin eine CH₂-Gruppe durch NR1 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl, (C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
n 0, 1 oder 2;
sowie deren physiologisch verträglichen Salze.

4. Verbindungen der Formel I, einem oder mehreren der Ansprüche 1 bis 3, **dadurch gekennzeichnet, dass** darin bedeuten
R1, R2 unabhängig voneinander H, Benzylaminocarbonyl, Benzoylamino, wobei die Benzylaminocarbonyl- und Benzoylaminoreste ein- oder mehrfach substituiert sein können mit F, Cl, Br, I, OH, CF₃, NO₂, CN, OCF₃, O-(C₁-C₆)-Alkyl, (C₁-C₆)-Allcyl, NH₂, NH(C₁-C₆)-Alkyl, N((C₁-C₆)-Alkyl)₂, SO₂-CH₃, COOH, COO-(C₁-C₆)-Alkyl, CONH₂;
wobei nicht beide Reste R1 oder R2 Wasserstoff entsprechen können;
R3 (C₂-C₁₀)-Alkenyl, Benzyl, wobei dder Benzylrest ein- oder mehrfach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Akyl, N((C₁-C₆)-Alkyl)₂, SO₂-CH₃, COOH, COO-(C₁-C₆)-Alkyl, CONH₂;
R4 und R5 bilden zusammen eine 3-5-gliedrige Alkylenkette, worin eine CH₂-Gruppe durch NH ersetzt ist, wobei die Alkylgruppen ein- oder mehrfach mit F, Cl, Br oder I substituiert sein können;
R1 H, (C₁-C₆)-Alkyl, (C₃-C₈)-Cycloalkyl, (C₁-C₄)-Alkylen-Aryl oder (C₁-C₄)-Alkylen-Heterocyclyl;
n 0;
sowie deren physiologisch verträglichen Salze.

5. Verbindungen gemäß einem oder mehreren der Ansprüche 1 bis 4 zur Anwendung als Arzneimittel.

6. Arzneimittel enthaltend eine oder mehrere der Verbindungen gemäß einem oder mehreren der Ansprüche 1 bis 4.

7. Arzneimittel enthaltend eine oder mehrere der Verbindungen gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alfa Agonisten, PPAR alfa/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-hibibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

9. Verwendung der Verbindungen gemäß einem oder mehreren der Ansprüche 1 bis 4 zur Herstellung eines Medikamentes zur Blutzuckersenkung.

10. Verwendung der Verbindungen gemäß einem oder mehreren der Ansprüche 1 bis 4 zur Herstellung eines Medikamentes zur Behandlung des Typ II Diabetes.

11. Verwendung der Verbindungen gemäß einem oder mehreren der Ansprüche 1 bis 4 zur Herstellung eines Medikamentes zur Behandlung von Lipid- und Kohlenhydratstoffwechselstörungen.

12. Verwendung der Verbindungen gemäß einem oder mehreren der Ansprüche 1 bis 4 zur Herstellung eines Medikaments zur Behandlung arteriosklerotischer Erscheinungen.

13. Verwendung der Verbindungen gemäß einem oder mehreren der Ansprüche 1 bis 4 zur Herstellung eines Medikaments zur Behandlung von Insulin Resistenz.

14. Verfahren zur Herstellung eines Arzneimittels enthaltend eine oder mehrere der Verbindungen gemäß einem oder mehreren der Ansprüche 1 bis 4, **dadurch gekennzeichnet, dass** der Wirkstoff mit einem pharmazeutisch geeigneten Träger vermischt wird und diese Mischung in eine für die Verabreichung geeignete Form gebracht wird.

15. Verwendung der Verbindungen der Formel I, 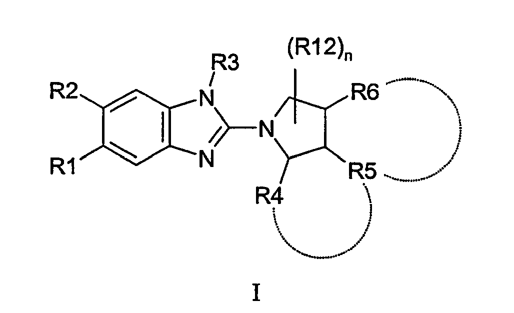 worin bedeuten
R1, R2 unabhängig voneinander H, CONR2OR21, NR22COR23 oder NR24R25;
R20, R21 unabhängig voneinander H, (C₁-C₁₀)-Alkyl, (C₃-C₁₀)-Cycloalkyl, (C₂-C₁₀)Alkenyl, (C₂-C₁₀)-Alkinyl, (C₆-C₁₀)-Aryl, Heterocyclyl, (C₁-C₆)-Alkylen-(C₆-C₁₀)-Aryl oder (C₁-C₆)-Akylen-(C₆-C₁₀)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R22, R23 unabhängig voneinander H, (C₁-C₁₀)-Alkyl, (C₃-C₁₀)-Cycloalkyl, (C₂-C₁₀)-Allcenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocylyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Akyl oder S(O)₂-(C₁-C₆)-Alkyl;
R24, R25 unabhängig voneinander H, (C₁-C₁₀)-Alkyl, (C₃-C₁₀)-Cycloalkyl, (C₂-C₁₀)-Alkenyl, (C₂-C₁₀)-Alkinyl, (C₆-C₁₀)-Aryl, Heterocyclyl, (C₁-C₆)-Alkylen-(C₆-C₁₀)-Aryl, (C₁-C₆)-Alkylen-(C₆-C₁₀)-Heterocyclyl oder S(O)₂-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, NO₂, SH, OH, (C₁-C₆)-Alkyl, O-(C₁-C₆)-Alkyl, S-(C₁-C₆)-Alkyl, S(O)-(C₁-C₆)-Alkyl oder S(O)₂-(C₁-C₆)-Alkyl;
R3 unabhängig voneinander H, (C₁-C₁₀)-Alkyl, (C₃-C₁₀)-Cycloalkyl, (C₂-C₁₀)-Alkenyl, (C₂-C₁₀)-Alkyl, (C₆-C₁₀)-Aryl oder Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, NO₂, SH, OH, (C₁-C₆)-Alkyl, -CF₃, -OCF₃, -SCF₃, (C₂-C₆)-Alkenyl, (C₂-C₆)-Alkinyl, OR7, OP(O)(OR7)₂, NR7R8, NR7CONR7R8, COR7, OCOR7, OCOOR7, COOR7, CONR7R8, OCONR7R8, (C₁-C₆)-Alkylen-OR7, (C₁-C₆)-Alkylen-NR7R8, (C₁-C₆)-Alkylen-NR7S(O)₂R7, (C₁-C₆)-Alkylen-SR7, (C₁-C₆)-Alkylen-S(O)R7, (C₁-C₆)-Alkylen-S(O)₂R7, (C₁-C₆)-Alkylen-S(O)₂NR7R8, (C₁-C₆)-Alkylen-COR7, (C₁-C₆)-Alkylen-COOR7, (C₁-C₆)-Alkylen-CONR7R8, SR7, S(O)R7, S(O)₂R7, S(O)₂NR7R8, NR7S(O)₂R7, (C₁-C₆)-Alkylen-(C₃-C₁₀)-Cycloalkyl, (C₁-C₆)-Alkylen-(C₆-C₁₀)-Aryl, (C₁-C₆)-Alkylen-Heterocyclyl, (C₃-C₁₀)-Cycloalkyl, (C₆-C₁₀)-Aryl oder Heterocyclyl;
R7, R8 unabhängig voneinander H, (C₁-C₆)-Alkyl, -CF₃, (C₃-C₁₀)-Cycloalkyl, (C₆-C₁₀)-Aryl, Heterocyclyl, (C₁-C₆)-Alkylen-CONR9R10, CONR9R10, (C₁-C₆)-Alkylen-COOR9, COOR9, COR9, (C₁-C₆)-Alkylen-COR9, (C₁-C₆₎-Alkylen-OR9, (C₁-C₆)-Alkylen-NR9R10, (C₁-C₆)-Alkylen-SR9, (C₁-C₆)-Alkylen-S(O)R9, (C₁-C₆)-Alkylen-S(O)₂R9, S(O)R9, S(O)₂R9, (C₁-C₄)-Akylen-(C₆-C₁)-Aryl oder (C₁-C₄)-Alkylen-Heterocyclyl;
R9, R10 unabhängig voneinander H, (C₁-C₆)-Alkyl, (C₁-C₆)-Alkylen-(C₆-C₁₀)-Aryl, -(C₆-C₁₀)-Aryl, Heterocyclyl oder (C₁-C₆)-Alkylen-Heterocyclyl;
R4 und R5 bilden zusammen eine 3-5-gliedrige Alkylenkette, worin eine CH₂-Gruppe durch NR11 ersetzt ist, wobei R6 gleich H oder R12 ist, oder
R5 und R6 bilden zusammen eine 3 bis 5 gliedrige Alkylenkette, worin eine CH₂-Gruppe durch NR11 ersetzt ist, wobei R4 gleich H oder R12 ist; wobei die 3 bis 5 gliedrige Alkylenkette jeweils ein- oder mehrfach substituiert sein kann mit F, Cl, Br, I, (C₁-C₆)-Alkyl, (C₃-C₈)-Cycloalkyl, NH₂, NH(C₁-C₆)-Alkyl, NH(C₃-C₇)-Cycloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
R11 H, (C₁-C₆)-Alkyl₂(C₃-C₈)-Cycloalkyl, (C₁-C₄)-Alkylen-Aryl oder (C₁-C₄)-Alkylen-Heterocyclyl;
R12 F, Cl, Br, I, (C₁-C₆)-Alkyl, (C₃-C₈)-Cyloalkyl, NH₂, NH(C₁-C₆)-Alkyl, NH(C₃-C₇)-Cyloalkyl, N((C₁-C₆)-Alkyl)₂ oder O-(C₁-C₆)-Alkyl, wobei die Alkylgruppen ein- oder mehrfach mit F, Cl, Br oder I substituiert sein können;
n 0, 1 oder 2;
sowie derer physiologisch verträglichen Salze zur Herstellung eines Medikaments zur Blutzuckersenkung.

## Claims

1. A compound of the formula I 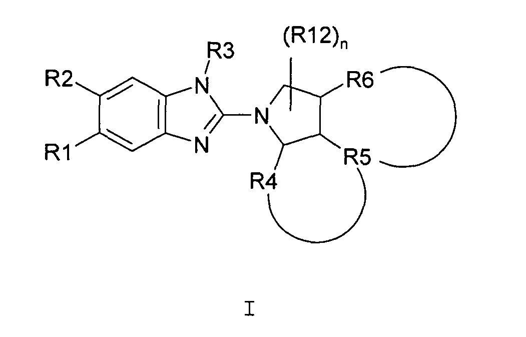 in which
R1, R2 are each independently H, CONR20R21, NR22COR23 or NR24R25, where the two R1 and R2 radicals cannot both be hydrogen;
R20, R21 are each independently H, (C₁-C₁₀) -alkyl, (C₃-C₁₀) -cycloalkyl, (C₂-C₁₀) -alkenyl, (CrC₁₀)-alkynyl, (C₆-C₁₀)-aryl, heterocyclyl, (C₁-C₆)-alkylene-(C₆-C₁₀)-aryl or (C₁-C₆)-alkylene-(C₆-C₁₀) -heterocyclyl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O-(C₁-C₆)-alkyl, S-(C₁-C₆)-alkyl, S(O)-(C₁-C₆)-alkyl or S(O)₂-(C₁-C₆)-alkyl;
excluding the definition of phenyl fused to nitrogen-containing 5-membered heteroaromatic rings for R20 and R21;
R22, R23 are each independently H, (C₁-C₁₀)-alkyl, (C₃-C₁₀)-cycloalkyl, (C₂-C₁₀)-alkenyl, (C₂-C₁₀)-alkynyl, (C₆-C₁₀)-aryl, heterocyclyl, (C₁-C₆)-alkylene-(C₆-C₁₀)-aryl, (C₁-C₆)-alkylene-(C₆-C₁₀)-heterocyclyl or S(O)₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O-(C₁-C₆-alkyl, S-(C₁-C₆)-alkyl, S(O)-(C₁-C₆)-alkyl or S(O)₂-(C₁-C₆)-alkyl;
R24, R25 are each independently H, (C₁-C₁₀)-alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene-(C₆-C₁₀)-aryl, (C₁-C₆)-alkylene- (C₆-C₁₀)-heterocyclyl or S(O)₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O- (C₁-C₆)-alkyl, S-(C₁-C₆)-alkyl, S(O)-(C₁-C₆)-alkylor S(O)₂-(C₁-C₆)-alkyl;
R3 are each independently H, (C₁-C₁₀)-alkyl, (C₃-C₁₀) -cycloalkyl, (C₂-C₁₀) -alkenyl, (C₂-C₁₀)-alkynyl, (C₆-C₁₀)-aryl or heterocyclyl, where the alkyl, cycloalkyl, alkenyl, alkynyl, aryl and heterocyclyl radicals may be mono- or polysubstituted by F, Cl, Br, I, CN, NO₂, SH, OH, (C₁-C₆)-alkyl, -CF₃, -OCF₃, -SCF₃, (C₂-C₆)-alkenyl, (C₂-C₆)-alkynyl, OR7, OP(O) (OR7)₂, NR7R8, NR7CONR7R8, COR7, OCOR7, OCOOR7, COOR7, CONR7R8, OCONR7R8, (C₁-C₆)-alkylene-OR7, (C₁-C₆)-alkylene-NR7R8, (C₁-C₆)-alkylene-NR7S (O)₂R7, (C₁-C₆) -alkylene-SR7, (C₁-C₆)-alkylene-S(O)R7, (C₁-C₆)-alkylene-S (O)₂R7, (C₁-C₆)-alkylene-S (O)₂NR7R8, (C₁-C₆) -alkylene-COR7, (C₁-C₆)-alkylene-COOR7, (C₁-C₆)-alkylene-CONR7R8, SR7, S(O)R7, S(O)₂R7, S(O)₂NR7R8, NR7S(O)₂R7, (C₁-C₆) -alkylene- (C₃-C₁₀)-cycloalkyl, (C₁-C₆) -alkylene- (C₆-C₁₀) -aryl, (C₁-C₆) -alkylene-heterocyclyl, (C₃-C₁₀)-cycloalkyl, (C₆-C₁₀)-aryl or heterocyclyl;
excluding the definition of unsubstituted or substituted piperidin-4-yl for R3;
R7, R8 are each independently H, (C₁-C₆) -alkyl, -CF₃, (C₃-C₁₀)-cycloalkyl, (C₆-C₁₀)-aryl, heterocyclyl, (C₁-C₆)-alkylene-CONR9R10, CONR9R10, (C₁-C₆)-alkylene-COOR9, COOR9, COR9, (C₁-C₆)-alkylene-COR9, (C₁-C₆)-alkylene-OR9, (C₁-C₆)-alkylene-NR9R10, (C₁-C₆)-alkylene-SR9, (C₁-C₆)-alkylene-S(O)R9, (C₁-C₆)-alkylene-S(O)₂R9, S(O)R9, S(O)₂R9, (C₁-C₄) -alkylene-(C₆-C₁₀)-aryl or (C₁-C₄) -alkylene-heterocyclyl;
R9, R10 are each independently H, (C₁-C₆)-alkyl, (C₁-C₆) -alkylene- (C₆-C₁₀)-aryl,
-(C₆-C₁₀)-aryl, heterocyclyl or (C₁-C₆)-alkylene-heterocyclyl;
R4 and R5 together form a 3-5-membered alkylene chain in which one CH₂ group has been replaced by NR11 where R6 is H or R12, or
R5 and R6 together form a 3- to 5-membered alkylene chain in which one CH₂ group has been replaced by NR11 where R4 is H or R12; where the 3- to 5-membered alkylene chain may in each case be mono- or polysubstituted by F, Cl, Br, I, (C₁-C₆)-alkyl, (C₃-C₈)-cycloalkyl, NH₂, NH(C₁-C₆)-alkyl, NH(C₃-C₇)-cycloalkyl, N((C₁-C₆)-alkyl)₂ or O-(C₁-C₆)-alkyl, where the alkyl groups may be mono- or polysubstituted by F, Cl, Br or I;
R11 is H, (C₁-C₆) -alkyl, (C₃-C₈)-cycloalkyl, (C₁-C₄)-alkylene-aryl or (C₁-C₄)-alkylene-heterocyclyl;
R12 is F, Cl, Br, I, (C₁-C₆) -alkyl, (C₃-C₈)-cycloalkyl, NH₂, NH(C₁-C₆) -alkyl, NH(C₃-C₇)-cycloalkyl, N((C₁-C₆) -alkyl) ₂ or O- (C₁-C₆)-alkyl, where the alkyl groups may be mono- or polysubstituted by F, Cl, Br or I;
n is 0, 1 or 2;
excluding compounds in which the radicals are simultaneously defined as follows:
R1 or R2 is CONR20R21 and R3 is hydrogen or CH₃;
and physiologically compatible salts thereof.

2. A compound of the formula I as claimed in claim 1, wherein
R1, R2 are each independently H, CONR20R21, NR22COR23 or NR24R25, where the two R1 and R2 radicals cannot both be hydrogen;
R20, R21 are each independently H, (C₁-C₁₀) -alkyl, (C₃-C₁₀) -cycloalkyl, (C₂-C₁₀)-alkenyl, (C₂-C₁₀)-alkynyl, (C₆-C₁₀)-aryl, heterocyclyl, (C₁-C₆)-alkylene- (C₆-C₁₀)-aryl or (C₁-C₆)-alkylene- (C₆-C₁₀)-heterocyclyl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O-(C₁-C₆)-alkyl, S-(C₁-C₆)-alkyl, S(O)-(C₁-C₆)-alkyl or S(O)₂- (C₁-C₆)-alkyl;
excluding the definition of phenyl fused to nitrogen-containing 5-membered heteroaromatic rings for R20 and R21;
R22, R23 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀) -aryl, (C₁-C₆) -alkylene- (C₆-C₁₀) -heterocyclyl or S (O) ₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆) -alkyl, O- (C₁-C₆)-alkyl, S-(C₁-C₆)-alkyl, S(O)-(C₁-C₆)-alkyl or S (O)2- (C₁-C₆) -alkyl;
R24, R25 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀)-aryl, heterocyclyl, (C₁-C₆)-alkylene- (C₆-C₁₀) -aryl, (C₁-C₆) -alkylene- (C₆-C₁₀) -heterocyclyl or S(O)₂-aryl, where the alkyl, cycloalkyl, alkenyl, alkynyl, alkylene, aryl and heterocyclyl radicals may be mono- or polysubstituted by F, Cl, Br, I, CN, NO, SH, OH, (C₁-C₆) -alkyl, O-(C₁-C₆)-alkyl, S-(C₁-C₆)-alkyl, S(O)-(C₁-C₆)-alkyl or S(O)₂- (C₁-C₆)-alkyl;
R3 is (C₂-C₁₀) -alkyl, (C₃-C₁₀) -cycloalkyl, (C₂-C₁₀) -alkenyl, (C₂-C₁₀) -alkynyl, - (C₆-C₁₀) -aryl or heterocyclyl, where the alkyl, cycloalkyl, alkenyl, alkynyl, aryl and heterocyclyl radicals may be mono- or polysubstituted by F, Cl, Br, I, CN, NO₂, SH, OH, (C₁-C₆) -alkyl, -CF₃, -OCF₃, -SCF₃, (C₂-C₆) -alkenyl, (C₂-C₆)-alkynyl, OR7, OP(O)(OR7)₂, NR7R8, NR7CONR7R8, COR7, OCOR7, OCOOR7, COOR7, CONR7R8, OCONR7R8, (C₁-C₆) -alkylene-OR7, (C₁-C₆)-alkylene-NR7R8, (C₁-C₆) -alkylene-NR7S (O)₂R7, (C₁-C₆)-alkylene-SR7, (C₁-C₆) -alkylene-S(O)R7, (C₁-C₆)-alkylene-S(O)₂R7, (C₁-C₆)-alkylene-S(O)₂NR7R8, (C₁-C₆)-alkylene-COR7, (C₁-C₆)-alkylene-COOR7, (C₁-C₆)-alkylene-CONR7R8, SR7, S (O) R7, S(O)₂R7, S(O)₂NR7R8, NR7S(O)₂R7, (C₁-C₆) -alkylene- (C₃-C₁₀) -cycloalkyl, (C₁-C₆)-alkylene- (C₆-C₁₀) -aryl, (C₁-C₆) -alkylene-heterocyclyl, (C₃-C₁₀) -cycloalkyl, (C₆-C₁₀)-aryl or heterocyclyl;
excluding
the definition of unsubstituted or substituted piperidin-4-yl for R3;
R7, R8 are each independently H, (C₁-C₆) -alkyl, -CF₃, (C₃-C₁₀) -cycloalkyl, (C₆₋C₁₀) -aryl, heterocyclyl, (C₁-C₆)-alkylene-CONR9R10, CONR9R10, (C₁-C₆)-alkylene-COOR9, COOR9, COR9, (C₁-C₆) -alkylene-COR9, (C₁-C₆) -alkylene-OR9, (C₁-C₆) -alkylene-NR9R10, (C₁-C₆) -alkylene-SR9, (C₁-C₆) -alkylene-S (O) R9, (C₁-C₆) -alkylene-S (O) ₂R9, S (O) R9, S (O) ₂R9, (C₁-C₄) -alkylene- (C₆-C₁₀) -aryl or (C₁-C₄) -alkylene-heterocyclyl;
R9, R10 are each independently H, (C₁-C₆) -alkyl, (C₁-C₆) -alkylene- (C₆-C₁₀)-aryl,
-(C₆-C₁₀)-aryl, heterocyclyl or (C₁-C₆)-alkylene-heterocyclyl;
R4 and R5 together form a 3-5-membered alkylene chain in which one CH₂ group has been replaced by NR11 where R6 is H or R12, or
R5 and R6 together form a 3- to 5-membered alkylene chain in which one CH₂ group has been replaced by NR11 where R4 is H or R12; where the 3- to 5-membered alkylene chain may in each case be mono- or polysubstituted by F, Cl, Br, I, (C₁-C₆)-alkyl, (C₃-C₈) -cycloalkyl, NH₂, NH(C₁-C₆)-alkyl, NH(C₃-C₇)-cycloalkyl, N((C₁-C₆)-alkyl)₂ or O-(C₁-C₆)-alkyl, where the alkyl groups may be mono- or polysubstituted by F, Cl, Br or I;
R11 is H, (C₁-C₆) -alkyl, (C₃-C₈) -cycloalkyl, (C₁-C₄) -alkylene-aryl or (C₁-C₄) -alkylene-heterocyclyl;
R12 is F, Cl, Br, I, (C₁-C₆) -alkyl, (C₃-C₈)-cycloalkyl, NH₂, NH(C₁-C₆)-alkyl, NH(C₃-C₇)-cycloalkyl, N((C₁-C₆)-alkyl)₂ or O- (C₁-C₆)-alkyl, where the alkyl groups may be mono- or polysubstituted by F, Cl, Br or I;
n is 0, 1 or 2;
and physiologically compatible salts thereof.

3. A compound of the formula I as claimed in claim 1 or 2, wherein
R1, R2 are each independently H, CONR20R21, NR22COR23 or NR24R25, where the two R1 and R2 radicals cannot both be hydrogen;
R20, R21 are each independently H, (C₁-C₁₀)-alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀) -aryl or (C₁-C₆)-alkylene-(C₆-C₁₀) -heterocyclyl , where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O- (C₁-C₆)-alkyl, S-(C₁-C₆)-alkyl, S(O)-(C₁-C₆)-alkyl or S(O)₂-(C₁-C₆)-alkyl;
excluding
the definition of phenyl fused to nitrogen-containing 5-membered heteroaromatic rings for R20 and R21;
R22, R23 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀)-aryl, heterocyclyl, (C₁-C₆)-alkylene- (C₆-C₁₀) -aryl, (C₁-C₆)-alkylene- (C₆-C₁₀) -heterocyclyl or S (O) ₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆) -alkyl, O-(C₁-C₆)-alkyl, S-(C₁-C₆)-alkyl, S(O)-(C₁-C₆)-alkyl or S (O) ₂- (C₁-C₆) -alkyl;
R24, R25 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀) -aryl, (C₁-C₆) -alkylene- (C₆-C₁₀) -heterocyclyl or S (O) ₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O-(C₁-C₆)-alkyl, S-(C₁-C₆)-alkyl, S(O)-(C₁-C₆)-alkyl or S (O) ₂- (C₁-C₆) -alkyl ;
R3 is (C₂C₁₀)-alkyl, (C₃-C₁₀) -cycloalkyl, (C₂-C₁₀) -alkenyl, (C₂-C₁₀) -alkynyl, (C₆-C₁₀) -aryl or heterocyclyl, where the alkyl, cycloalkyl, alkenyl, alkynyl, aryl and heterocyclyl radicals may be mono- or polysubstituted by F, Cl, Br, I, CN, NO₂, SH, OH, (C₁-C₆) -alkyl, -CF₃, -OCF₃, -SCF₃, (C₂-C₆) -alkenyl, (C₂-C₆)-alkynyl, OR7, OP(O)(OR7)₂, NR7R8, NR7CONR7R8, COR7, OCOR7, OCOOR7, COOR7, CONR7R8, OCONR7R8, (C₁-C₆) -alkylene-OR7, (C₁-C₆)-alkylene-NR7R8, (C₁-C₆) -alkylene-NR7S (O)₂R7, (C₁-C₆) -alkylene-SR7, (C₁-C₆) -alkylene-S (O) R7, (C₁-C₆) -alkylene-S (O) ₂R7, (C₁-C₆) -alkylene-S(O)₂NR7R8, (C₁-C₆) -alkylene-COR7, (C₁-C₆)-alkylene-COOR7, (C₁-C₆)-alkylene-CONR7R8, SR7, S (O) R7, S(O)₂R7, S(O)₂NR7R8, NR7S(O)₂R7, (C₁-C₆) -alkylene- (C₃-C₁₀) -cycloalkyl, (C₁-C₆)-alkylene- (C₆-C₁₀) -aryl, (C₁-C₆) -alkylene-heterocyclyl, (C₃-C₁₀) -cycloalkyl, (C₆-C₁₀)-aryl or heterocyclyl;
excluding
the definition of unsubstituted or substituted piperidin-4-yl for R3;
R7, R8 are each independently H, (C₁-C₆) -alkyl, -CF₃, (C₃-C₁₀) -cycloalkyl, (C₆-C₁₀) -aryl, heterocyclyl, (C₁-C₆)-alkylene-CONR9R10, CONR9R10, (C₁-C₆)-alkylene-COOR9, COOR9, COR9, (C₁-C₆ -alkylene-COR9, (C₁-C₆)-alkylene-OR9, (C₁-C₆)-alkylene-NR9R10, (C₁-C₆)-alkylene-SR9, (C₁-C₆)-alkylene-S(O)R9, (C₁-C₆)-alkylene-S (O) ₂R9, S (O) R9, S (O) ₂R9, (C₁-C₄) -alkylene- (C₆-C₁₀) -aryl or (C₁-C₄) -alkylene-heterocyclyl;
R9, R10 are each independently H, (C₁-C₆) -alkyl, (C₁-C₆) -alkylene- (C₆-C₁₀) -aryl, - (C₆-C₁₀) -aryl, heterocyclyl or (C₁-C₆)-alkylene-heterocyclyl;
R4 and R5 together form a 3-5-membered alkylene chain in which one CH₂ group has been replaced by NR11 where R6 is H or R12, or
R5 and R6 together form a 3- to 5-membered alkylene chain in which one CH₂ group has been replaced by NR11 where R4 is H or R12; where the 3- to 5-membered alkylene chain may in each case be mono- or polysubstituted by F, Cl, Br, I, (C₁-C₆) -alkyl, (C₃-C₈) -cycloalkyl, NH₂, NH(C₁-C₆)-alkyl, NH (C₃-C₇) -cycloalkyl, N (C₁-C₆)-alkyl)₂ or O- (C₁-C₆) -alkyl, where the alkyl groups may be mono- or polysubstituted by F, Cl, Br or I;
R11 is H, (C₁-C₆) -alkyl, (C₃-C₈) -cycloalkyl, (C₁-C₄) -alkylene-aryl or (C₁-C₄)-alkylene-heterocyclyl;
R12 is F, Cl, Br, I, (C₁-C₆) -alkyl, (C₃-C₈)-cycloalkyl, NH₂, NH (C₁-C₆) -alkyl, NH(C₃-C₇)-cycloalkyl, N((C₁-C₆)-alkyl) or O-(C₁-C₆)-alkyl, where the alkyl groups may be mono- or polysubstituted by F, Cl, Br or I;
n is 0, 1 or 2;
and physiologically compatible salts thereof.

4. A compound of the formula I as claimed in one or more of claims 1 to 3, wherein
R1, R2 are each independently H, benzylaminocarbonyl, benzoylamino, where the benzylaminocarbonyl and benzoylamino radicals may be mono- or polysubstituted by F, Cl, Br, I, OH, CF₃, NO₂, CN, OCF₃, O- (C₁-C₆) -alkyl, (C₁-C₆) -alkyl, NH₂, NH (C₁-C₆) -alkyl, N((C₁-C₆)-alkyl)₂, SO₂-CH₃, COOH, COO- (C₁-C₆) -alkyl, CONH₂;
where the two R1 and R2 radicals cannot both be hydrogen;
R3 is (C₂-C₁₀)-alkenyl, benzyl, where the benzyl radical may be mono- or polysubstituted by F, Cl, Br, I, OH, CF₃, NO₂, CN, OCF₃, O- (C₁-C₆)-alkyl, (C₁-C₆) -alkyl, NH₂, NH(C₁-C₆) -alkyl, N((C₁-C₆)-alkyl)₂, SO₂-CH₃, COOH, COO-(C₁-C₆)-alkyl, CONH₂;
R4 and R5 together form a 3-5-membered alkylene chain in which one CH₂ group has been replaced by NH, where the alkyl groups may be mono- or polysubstituted by F, Cl, Br or I;
R11 is H, (C₁-C₆) -alkyl, (C₃-C₈) -cycloalkyl, (C₁-C₄) -alkylene-aryl or (C₁-C₄) -alkylene-heterocyclyl;
n is 0;
and physiologically compatible salts thereof.

5. A compound as claimed in one or more of claims 1 to 4 for use as a medicament.

6. A medicament comprising one or more of the compounds as claimed in one or more of claims 1 to 4.

7. A medicament comprising one or more of the compounds as claimed in one or more of claims 1 to 4 and at least one further active ingredient.

8. A medicament as claimed in claim 7, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin antagonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotonin and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptin, doprexin), lipase/amylase inhibitors, PPAR modulators, RXR modulators or TR-β agonists or amphetamines.

9. The use of the compounds as claimed in one or more of claims 1 to 4 for producing a medicament for lowering blood sugar.

10. The use of the compounds as claimed in one or more of claims 1 to 4 for producing a medicament for treating type II diabetes.

11. The use of the compounds as claimed in one or more of claims 1 to 4 for producing a medicament for treating lipid and carbohydrate metabolism disorders.

12. The use of the compounds as claimed in one or more of claims 1 to 4 for producing a medicament for treating arteriosclerotic manifestations.

13. The use of the compounds as claimed in one or more of claims 1 to 4 for producing a medicament for treating insulin resistance.

14. A process for producing a medicament comprising one or more of the compounds as claimed in one or more of claims 1 to 4, which comprises mixing the active ingredient with a pharmaceutically suitable carrier and bringing this mixture into a form suitable for administration.

15. The use of the compounds of the formula I 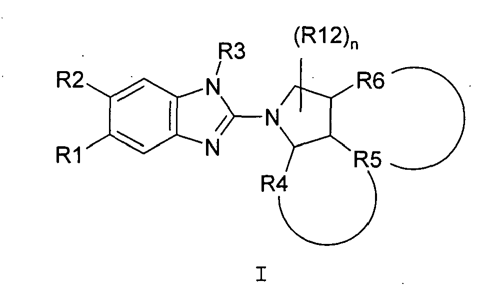 in which
R1, R2 are each independently H, CONR20R21, NR22COR23 or NR24R25;
R20, R21 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀) -aryl or (C₁-C₆) -alkylene- (C₆-C₁₀)-heterocyclyl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆) -alkyl, O- (C₁-C₆) -alkyl, S-(C₁-C₆)-alkyl, S (O) - (C₁-C₆) -alkyl or S(O)₂-(C₁-C₆)-alkyl;
R22, R23 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀)-aryl, (C₁-C₆) -alkylene- (C₆-C₁₀) -heterocyclyl or S(O)₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆) -alkyl, O-(C₁-C₆)-alkyl, S-(C₁-C₆)-alkyl, S (O) - (C₁-C₆) -alkyl or S (O) ₂- (C₁-C₆) -alkyl;
R24, R25 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀) -aryl, heterocyclyl, (C₁-C₆)-alkylene- (C₆-C₁₀) -aryl, (C₁-C₆) -alkylene- (C₆-C₁₀) -heterocyclyl or S (O) ₂-aryl, where the alkyl, cycloalkyl, alkenyl, alkynyl, alkylene, aryl and heterocyclyl radicals may be mono- or polysubstituted by F, Cl, Br, I, CN, NO₂, SH, OH, (C₁-C₆)-alkyl, O- (C₁-C₆)-alkyl, S-(C₁-C₆) -alkyl, S(O)-(C₁-C₆)-alkyl or S(O)₂-(C₁-C₆) -alkyl;
R3 are each independently H, (C₁-C₁₀) -alkyl, (C₃-C₁₀) -cycloalkyl, (C₂-C₁₀) -alkenyl, (C₂-C₁₀)-alkynyl, (C₆-C₁₀)-aryl or heterocyclyl, where the alkyl, cycloalkyl, alkenyl, alkynyl, aryl and heterocyclyl radicals may be mono- or polysubstituted by F, Cl, Br, I, CN, NO₂, SH, OH, (C₁-C₆) -alkyl, -CF₃, -OCF₃, -SCF₃, (C₂-C₆)-alkenyl, (C₂-C₆)-alkynyl, OR7, OP (O) (OR₇)₂, NR7R8, NR7CONR7R8, COR7, OCOR7, OCOOR7, COOR7, CONR7R8, OCONR7R8, (C₁-C₆)-alkylene-OR7, (C₁-C₆) -alkylene-NR7R8, (C₁-C₆)-alkylene-NR7S (O)₂R7, (C₁-C₆) -alkylene-SR7, (C₁-C₆)-alkylene-S(O)R7, (C₁-C₆) -alkylene-S (O) ₂R7, (C₁-C₆) -alkylene-S (O) ₂NR7R8, (C₁-C₆) -alkylene-COR7, (C₁-C₆)-alkylene-COOR7, (C₁-C₆)-alkylene-CONR7R8, SR7, S(O)R7, S(O)₂R7, S(O)₂NR7R8, NR7S(O)₂R7, (C₁-C₆)-alkylene- (C₃-C₁₀)-cycloalkyl, (C₁-C₆) -alkylene- (C₆-C₁₀) -aryl, (C₁-C₆) -alkylene-heterocyclyl, (C₃-C₁₀)-cycloalkyl, (C₆-C₁₀)-aryl or heterocyclyl;
R7, R8 are each independently H, (C₁-C₆) -alkyl, -CF₃, (C₃-C₁₀) -cycloalkyl, (C₆-C₁₀) -aryl, heterocyclyl, (C₁-C₆)-alkylene-CONR9R10, CONR9R10, (C₁-C₆)-alkylene-COOR9, COOR9, COR9, (C₁-C₆) -alkylene-COR9, (C₁-C₆) -alkylene-OR9, (C₁-C₆) -alkylene-NR9R10, (C₁-C₆) -alkylene-SR9, (C₁-C₆) -alkylene-S (O) R9, (C₁-C₆)-alkylene-S(O)₂R9, S(O)R9, S(O)₂R9, (C₁-C₄)-alkylene-(C₆-C₁₀)-aryl or (C₁-C₄) -alkylene-heterocyclyl;
R9, R10 are each independently H, (C₁-C₆)-alkyl, (C₁-C₆) -alkylene- (C₆-C₁₀) -aryl, - (C₆-C₁₀) -aryl, heterocyclyl or (C₁-C₆)-alkylene-heterocyclyl;
R4 and R5 together form a 3-5-membered alkylene chain in which one CH₂ group has been replaced by NR11 where R6 is H or R12, or
R5 and R6 together form a 3- to 5-membered alkylene chain in which one CH₂ group has been replaced by NR11 where R4 is H or R12; where the 3- to 5-membered alkylene chain may in each case be mono- or polysubstituted by F, Cl, Br, I, (C₁-C₆) -alkyl, (C₃-C₈) -cycloalkyl, NH₂, NH (C₁-C₆)-alkyl, NH(C₃-C₇)-cycloalkyl, N((C₁-C₆)-alkyl)₂ or O-(C₁-C₆) -alkyl, where the alkyl groups may be mono- or polysubstituted by F, Cl, Br or I;
R11 is H, (C₁-C₆)-alkyl, (C₃-C₈)-cycloalkyl, (C₁-C₄) -alkylene-aryl or (C₁-C₄)-alkylene-heterocyclyl;
R12 is F, Cl, Br, I, (C₁-C₆) -alkyl, (C₃-C₈)-cycloalkyl, NH₂, NH(C₁-C₆) -alkyl, NH(C₃-C₇)-cycloalkyl, N((C₁-C₆)-alkyl)₂ or O-(C₁-C₆)-alkyl, where the alkyl groups may be mono- or polysubstituted by F, Cl, Br or I;
n is 0, 1 or 2;
and physiologically compatible salts thereof for producing a medicament for lowering blood sugar.

## Revendications

1. Composés de formule I 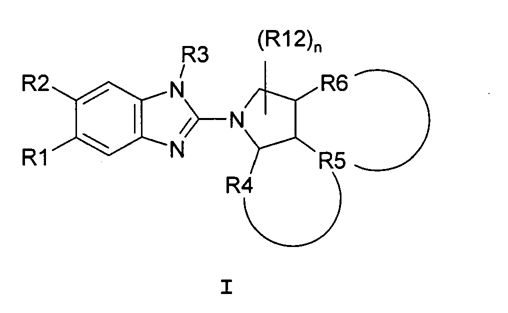 dans laquelle .
R1 et R2 représentent, indépendamment l'un de l'autre, un atome d'hydrogène, un groupe CONR20R21, un groupe NR22COR23 ou un groupe NR24R25, où les deux radicaux R1 ou R2 ne peuvent pas représenter un atome d'hydrogène ;
R20 et R21 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle ou un groupe (C₁-C₆) -alkylène- (C₆-C₁₀)-hétéro-cyclyle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O-(C₁-C₆)-alkyle, un groupe S-(C₁-C₆) -alkyle, un groupe S (O) - (C₁-C₆) -alkyle ou un groupe S (O) ₂- (C₁-C₆) -alkyle ;
la définition d'un groupe phényle condensé avec des noyaux hétéroaromatiques azotés à 5 chaînons étant exclue pour R20 et R21;
R22 et R23 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀) un groupe hétérocyclyle, un groupe (C₁-C₆) -alkylène- (C₆-C₁₀)-aryle, un groupe (C₁-C₆) -alkylène- (C₆-C₁₀) -hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S- (C₁-C₆)-alkyle, un groupe S (O) - (C₁-C₆) -alkyle ou un groupe S (O) ₂- (C₁-C₆) -alkyle ;
R24 et R25 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle, un groupe (C₁-C₆) -alkylène- (C₆-C₁₀) -hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S (O) ₂- (C₁-C₆) -alkyle ;
R3 représente indépendamment un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀) ou un groupe hétérocyclyle, où les radicaux alkyle, cycloalkyle, alcényle, alcynyle, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe -CF₃, -OCF₃, -SCF₃, un groupe alcényle en (C₂-C₆), un groupe alcynyle en (C₂-C₆), un groupe OR7, un groupe P(O) (OR7)₂, un groupe NR7R8, un groupe NR7CONR7R8, un groupe COR7, un groupe OCOR7, un groupe OCOOR7, un groupe COOR7, un groupe CONR7R8, un groupe OCONR7R8, un groupe (C₁-C₆)-alkylène-OR7, un groupe (C₁-C₆) -alkylène-NR7R8, un groupe (C₁-C₆) -alkylène-NR7S (O)₂R7, un groupe (C₁-C₆) -alkylène-SR7, un groupe (C₁-C₆)-alkylène-S (O) R7, un groupe (C₁-C₆)-alkylène-S(O) ₂R7, un groupe (C₁-C₆)-alkylène-S (O) ₂NR7R8, un groupe (C₁-C₆) -alkylène-COR7, un groupe (C₁-C₆) -alkylène-COOR7, un groupe (C₁-C₆) -alkylène-CONR7R8, un groupe SR7, un groupe S (O) R7, un groupe S (O)₂R7, un groupe S (O)₂NR7R8, un groupe NR7S (O)₂R7, un groupe (C₁-C₆) -alkylène- (C₃-C₁₀) -cycloalkyle, un groupe (C₁-C₆) -alkylène- (C₆-C₁₀) -aryle, un groupe (C₁-C₆)-alkylène-hétérocyclyle, un groupe cycloalkyle en (C₃-C₁₀), un groupe aryle en (C₆-C₁₀) ou un groupe hétéro-cyclyle ;
la définition d'un groupe pipéridin-4-yle non substitué ou substitué étant exclue pour R3 ;
R7 et R8 représentent, indépendamment l'un de l'autre, un atome d'hydrogène, un groupe alkyle en (C₁-C₆), un groupe -CF₃, un groupe cycloalkyle en (C₃-C₁₀), un groupe aryle en (C₆-C₁₀), un groupe hétérocyclyle, un groupe (C₁-C₆) -alkylène-CONR9R10, un groupe CONR9R10, un groupe (C₁-C₆) -alkylène-COOR9, un groupe COOR9, un groupe COR9, un groupe (C₁-C₆) -alkylène-COR9, un groupe (C₁-C₆)-alkylène-OR9, un groupe (C₁-C₆)-alkylène-NR9R10, un groupe (C₁-C₆)-alkylène-SR9, un groupe (C₁-C₆) -alkylène-S (O) R9, un groupe (C₁-C₆) -alkylène-S (O)_{z}R⁹, un groupe S(O) R9, un groupe S (O) ₂R9, un groupe (C₁-C₄)-alkylène- (C₆-C₁₀) -aryle ou un groupe (C₁-C₄)-alkylène-hétérocyclyle ;
R9 et R10 représentent, indépendamment l'un de l'autre, un atome d'hydrogène, un groupe alkyle en (C₁-C₆), un groupe (C₁-C₆)-alkylène- (C₆-C₁₀) -aryle, un groupe aryle en (C₆-C₁₀), un groupe hétérocyclyle ou un groupe (C₁-C₆) -alkylène-hétérocyclyle
R4 et R5 forment conjointement une chaîne alkylène à 3-5 chaînons, dans laquelle un groupe CH₂ est remplacé par un groupe NR11, où R6 représente un atome d'hydrogène ou un groupe R12, ou
R5 et R6 forment conjointement une chaîne alkylène à 3-5 chaînons, dans laquelle un groupe CH₂ est remplacé par un groupe NR11, où R4 représente un atome d'hydrogène ou un groupe R12 ; la chaîne alkylène à 3-5 chaînons pouvant être, dans chaque cas, mono- ou poly-substituée par un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH(C₃-C₇)-cycloalkyle, un groupe N((C₁-C₆) -alkyle) ou un groupe O-(C₁-C₆)-alkyle, où les groupes alkyle peuvent être mono- ou poly-substitués par un atome de fluor, un atome de chlore, un atome de brome ou un atome d'iode ;
R11 représente un atome d'hydrogène, un groupe alkyle en (C₁-C₆), un groupe cycloalkyle en (C₃-C₈), un groupe (C₁-C₄) -alkylène-aryle ou un groupe (C₁-C₄)-alkylène-hétérocyclyle ;
R12 représente un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH(C₃-C₇)-cycloalkyle, un groupe N ((C₁-C₆)-alkyle)₂ ou un groupe O- (C₁-C₆) -alkyle, où les groupes alkyle peuvent être mono- ou poly-substitués par un atome de fluor, un atome de chlore, un atome de brome ou un atome d'iode ;
n vaut 0, 1 ou 2 ;
à l'exclusion des composés dans lesquels les radicaux présentent simultanément les significations suivantes .
R1 ou R2 représentent un groupe CONR20R21 et R3 représente un atome d'hydrogène ou un groupe CH₃ ;
ainsi que leurs sels physiologiquement acceptables.

2. Composés de formule I selon la revendication 1, **caractérisés en ce que** :
R1 et R2 représentent, indépendamment l'un de l'autre, un atome d'hydrogène, un groupe CONR20R21, un groupe NR22COR23 ou un groupe NR24R25, où les deux radicaux R1 ou R2 ne peuvent pas représenter un atome d'hydrogène ;
R20 et R21 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle ou un groupe (C₁-C₆) -alkylène- (C₆-C₁₀) -hétéro-cyclyle, où les radicaux alkyle, cycloalkyle, alcényle, alcynyle, alkylène, aryle et hétérocyclyle peuvent être mono- ou poly-substitués par un atome de fluor,
un atome de chlore, un atome de brome, un atome d'iode, un groupe CN, un groupe NO₂, un groupe SH, un groupe OH, un groupe alkyle en (C₁-C₆), un groupe O-(C₁-C₆)-alkyle, un groupe S-(C₁-C₆) -alkyle, un groupe S (O) - (C₁-C₆) -alkyle ou un groupe S (O)₂- (C₁-C₆) -alkyle ;
la définition d'un groupe phényle condensé avec des noyaux hétéroaromatiques azotés à 5 chaînons étant exclue pour R20 et R21 ;
R22 et R23 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle, un groupe (C₁-C₆) -alkylène- (C₆-C₁₀) -hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S (O) ₂- (C₁-C₆) -alkyle ;
R24 et R25 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène-(C₆-C₁₀)-aryle, un groupe (C₁-C₆) -alkylène- (C₆-C₁₀) -hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S(O)₂-(C₁-C₆)-alkyle ;
R3 représente un groupe alkyle en (C₂-C₁₀), un groupe cycloalkyle en (C₃-C₁₀), un groupe alcényle en (C₂-C₁₀), un groupe alcynyle en (C₂-C₁₀), un groupe aryle en (C₆-C₁₀) ou un groupe hétérocyclyle, où les radicaux alkyle, cycloalkyle, alcényle, alcynyle, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe -CF₃, -OCF₃, -SCF₃, un groupe alcényle en (C₂-C₆), un groupe alcynyle en (C₂-C₆), un groupe OR7, un groupe OP(O)(OR7)₂, un groupe NR7R8, un groupe NR7CONR7R8, un groupe COR7, un groupe OCOR7, un groupe OCOOR7, un groupe COOR7, un groupe CONR7R8, un groupe OCONR7R8, un groupe (C₁-C₆) -alkylène-OR7, un groupe (C₁-C₆) -alkylène-NR7R8, un groupe (C₁-C₆)-alkylène-NR7S (O)₂R7, un groupe (C₁-C₆)-alkylène-SR7, un groupe (C₁-C₆)-alkylène-S (O) R7, un groupe (C₁-C₆) -alkylène-S(O)₂R7, un groupe (C₁-C₆) -alkylène-S(O)₂NR7R8, un groupe (C₁-C₆) -alkylène-COR7, un groupe (C₁-C₆) -alkylène-COOR7, un groupe (C₁-C₆)-alkylène-CONR7R8, un groupe SR7, un groupe S(O)R7, un groupe S(O)₂R7, un groupe S(O)₂NR7R8, un groupe NR7S(O)₂R7, un groupe (C₁-C₆) -alkylène- (C₃-C₁₀) -cycloalkyle, un groupe (C₁-C₆) -alkylène- (C₆-C₁₀) -aryle, un groupe (C₁-C₆)-alkylène-hétérocyclyle, un groupe cycloalkyle en (C₃-C₁₀), un groupe aryle en (C₆-C₁₀) ou un groupe hétéro-cyclyle ;
la définition d'un groupe pipéridin-4-yle non substitué ou substitué étant exclue pour R3 ;
R7 et R8 représentent, indépendamment l'un de l'autre, un atome d'hydrogène, un groupe alkyle en (C₁-C₆), un groupe -CF₃, un groupe cycloalkyle en (C₃-C₁₀), un groupe aryle en (C₆-C₁₀), un groupe hétérocyclyle, un groupe (C₁-C₆) -alkylène-CONR9R10, un groupe CONR9R10, un groupe (C₁-C₆) -alkylène-COOR9, un groupe COOR9, un groupe COR9, un groupe (C₁-C₆) -alkylène-COR9, un groupe (C₁-C₆)-alkylène-OR9, un groupe (C₁-C₆)-alkylène-NR9R10, un groupe (C₁-C₆)-alkylène-SR9, un groupe (C₁-C₆) -alkylène-S (O) R9, un groupe (C₁-C₆) -alkylène-S (O)₂R9, un groupe S (O) R9, un groupe S (O)₂ R9, un groupe (C₁-C₄)-alkylène- (C₆-C₁₀) -aryle ou un groupe (C₁-C₄)-alkylène-hétérocyclyle ;
R9 et R10 représentent, indépendamment l'un de l'autre, un atome d'hydrogène, un groupe alkyle en (C₁-C₆), un groupe (C₁-C₆)-alkylène- (C₆-C₁₀) -aryle, un groupe aryle en (C₆-C₁₀), un groupe hétérocyclyle ou un groupe (C₁-C₆)-alkylène-hétérocyclyle ;
R4 et R5 forment conjointement une chaîne alkylène à 3-5 chaînons, dans laquelle un groupe CH₂ est remplacé par un groupe NR11, où R6 représente un atome d'hydrogène ou un groupe R12, ou
R5 et R6 forment conjointement une chaîne alkylène à 3-5 chaînons, dans laquelle un groupe CH₂ est remplacé par un groupe NR11, où R4 représente un atome d'hydrogène ou un groupe R12 ; la chaîne alkylène à 3-5 chaînons pouvant être, dans chaque cas, mono- ou poly-substituée par un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH(C₃-C₇)-cycloalkyle, un groupe N ((C₁-C₆) -alkyle) ₂ ou un groupe O- (C₁-C₆)-alkyle, où les groupes alkyle peuvent être mono- ou poly-substitués par un atome de fluor, un atome de chlore, un atome de brome ou un atome d'iode ;
R11 représente un atome d'hydrogène, un groupe alkyle en (C₁-C₆), un groupe cycloalkyle en (C₃-C₈), un groupe (C₁-C₄) -alkylène-aryle ou un groupe (C₁-C₄)-alkylène-hétérocyclyle ;
R12 représente un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH(C₃-C₇)-cycloalkyle, un groupe N (C₁-C₆) -alkyle) ₂ ou un groupe O- (C₁-C₆) -alkyle, où les groupes alkyle peuvent être mono- ou poly-substitués par un atome de fluor, un atome de chlore, un atome de brome ou un atome d'iode ;
n vaut 0, 1 ou 2;
ainsi que leurs sels physiologiquement acceptables.

3. Composés de formule I selon la revendication 1 ou 2, **caractérisés en ce que**:
R1 et R2 représentent, indépendamment l'un de l'autre, un atome d'hydrogène, un groupe CONR20R21, un groupe NR22COR23 ou un groupe NR24R25, où les deux radicaux R1 ou R2 ne peuvent pas représenter un atome d'hydrogène ;
R20 et R21 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle ou un groupe (C₁-C₆) -alkylène- (C₆-C₁₀) -hétéro-cyclyle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆)-alkyle, un groupe S-(C₁-C₆)-alkyle, un groupe S (O) - (C₁-C₆) -alkyle ou un groupe S (O) ₂- (C₁-C₆) -alkyle ;
la définition d'un groupe phényle condensé avec des noyaux hétéroaromatiques azotés à 5 chaînons étant exclue pour R20 et R21 ;
R22 et R23 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle, un groupe (C₁-C₆) -alkylène- (C₆-C₁₀) -hétérocyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S (O) ₂- (C₁-C₆) -alkyle ;
R24 et R25 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆)-alkylène-(C₆-C₁₀)-aryle, un groupe (C₁-C₆)-alkylène-(C₆-C₁₀)-hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S (O) ₂- (C₁-C₆) -alkyle ;
R3 représente un groupe alkyle en (C₂-C₁₀), un groupe cycloalkyle en (C₃-C₁₀), un groupe alcényle en (C₂-C₁₀), un groupe alcynyle en (C₂-C₁₀), un groupe aryle en (C₆-C₁₀) ou un groupe hétérocyclyle, où les radicaux alkyle, cycloalkyle, alcényle, alcynyle, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe -CF₃, -OCF₃, -SCF₃, un groupe alcényle en (C₂-C₆), un groupe alcynyle en (C₂-C₆), un groupe OR7, un groupe OP(O)(OR7)₂, un groupe NR7R8, un groupe NR7CONR7R8, un groupe COR7, un groupe OCOR7, un groupe OCOOR7, un groupe COOR7, un groupe CONR7R8, un groupe OCONR7R8, un groupe (C₁-C₆) -alkylène-OR7, un groupe (C₁-C₆) -alkylène-NR7R8, un groupe (C₁-C₆) -alkylène-NR7S (O) ₂R7, un groupe (C₁-C₆) -alkylène-SR7, un groupe (C₁-C₆)-alkylène-S (O) R7, un groupe (C₁-C₆)-alkylène-S(O)₂R7, un groupe (C₁-C₆)-alkylène-S(O)₂NR7R8, un groupe (C₁-C₆)-alkylène-COR7, un groupe (C₁-C₆)-alkylène-COOR7, un groupe (C₁-C₆)-alkylène-CONR7R8, un groupe SR7, un groupe S(O)R7, un groupe S(O)₂R7, un groupe S(O)₂NR7R8, un groupe NR7S(O)₂R7, un groupe (C₁-C₆) -alkylène- (C₃-C₁₀) -cycloalkyle, un groupe (C₁-C₆)-alkylène-(C₆-C₁₀)-aryle, un groupe (C₁-C₆)-alkylène-hétérocyclyle, un groupe cycloalkyle en (C₃-C₁₀), un groupe aryle en (C₆-C₁₀) ou un groupe hétérocyclyle ;
la définition d'un groupe pipéridin-4-yle non substitué ou substitué étant exclue pour R3 ;
R7 et R8 représentent, indépendamment l'un de l'autre, un atome d'hydrogène, un groupe alkyle en (C₁-C₆), un groupe -CF₃, un groupe cycloalkyle en (C₃-C₁₀), un groupe aryle en (C₆-C₁₀), un groupe hétérocyclyle, un groupe (C₁-C₆)-alkylène-CONR9R10, un groupe CONR9R10, un groupe (C₁-C₆)-alkylène-COOR9, un groupe COOR9, un groupe COR9, un groupe (C₁-C₆) -alkylène-COR9, un groupe (C₁-C₆)-alkylène-OR9, un groupe (C₁-C₆)-alkylène-NR9Rl0, un groupe (C₁-C₆) -alkylène-SR9, un groupe (C₁-C₆) -alkylène-S (O) R9, un groupe (C₁-C₆) -alkylène-S (O) zR9, un groupe S(O)R9, un groupe S (O) ₂R9, un groupe (C₁-C₄)-alkylène- (C₆-C₁₀) -aryle ou un groupe (C₁-C₄)-alkylène-hétérocyclyle ;
R9 et R10 représentent, indépendamment l'un de l'autre, un atome d'hydrogène, un groupe alkyle en (C₁-C₆) un groupe (C₁-C₆)-alkylène-(C₆-C₁₀)-aryle, un groupe aryle en (C₆-C₁₀), un groupe hétérocyclyle ou un groupe (C₁-C₆) -alkylène-hétérocyclyle ;
R4 et R5 forment conjointement une chaîne alkylène à 3-5 chaînons, dans laquelle un groupe CH₂ est remplacé par un groupe NR11, où R6 représente un atome d'hydrogène ou un groupe R12, ou
R5 et R6 forment conjointement une chaîne alkylène à 3-5 chaînons, dans laquelle un groupe CH₂ est remplacé par un groupe NR11, où R4 représente un atome d'hydrogène ou un groupe R12 ; la chaîne alkylène à 3-5 chaînons pouvant être, dans chaque cas, mono- ou poly-substituée par un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH(C₃-C₇)-cycloalkyle, un groupe N((C₁-C₆) -alkyle) ₂ ou un groupe O-(C₁-C₆)-alkyle, où les groupes alkyle peuvent être mono- ou poly-substitués par un atome de fluor, un atome de chlore, un atome de brome ou un atome d'iode ;
R11 représente un atome d'hydrogène, un groupe alkyle en (C₁-C₆), un groupe cycloalkyle en (C₃-C₈), un groupe (C₁-C₄) -alkylène-aryle ou un groupe (C₁-C₄) -alkylène-hétérocyclyle ;
R12 représente un atome de fluor, un atome de chlore, un atome de brome, un atome d'iode, un groupe alkyle en (C₁-C₆), un groupe cycloalkyle en (C₃-C₈), un groupe NH₂, un groupe NH(C₁-C₆)-alkyle, un groupe NH (C₃-C₇)-cycloalkyle, un groupe N((C₁-C₆)-alkyle)₂ ou un groupe O- (C₁-C₆) -alkyle, où les groupes alkyle peuvent être mono- ou polysubstitués par un atome de fluor, un atome de chlore, un atome de brome ou un atome d'iode ;
n vaut 0, 1 ou 2 ;
ainsi que leurs sels physiologiquement acceptables.

4. Composés de formule I selon l'une ou plusieurs des revendications 1 à 3, **caractérisés en ce que** :
R1 et R2 représentent, indépendamment l'un de l'autre, un atome d'hydrogène, un groupe benzylaminocarbonyle, un groupe benzoylamino, où les radicaux benzylaminocarbonyle et benzoylamino peuvent être mono- ou polysubstitués par un atome de fluor, un atome de chlore, un atome de brome, un atome d'iode, un groupe OH, un groupe CF₃, un groupe NO₂, un groupe CN, un groupe OCF₃, un groupe O- (C₁-C₆) -alkyle, un groupe alkyle en (C₁-C₆) , un groupe NH₂, un groupe NH(C₁-C₆)-alkyle, un groupe N ((C₁-C₆)-alkyle)₂, un groupe SO₂-CH₃, un groupe COOH, un groupe COO- (C₁-C₆) -alkyle, un groupe CONH₂ ;
où les deux radicaux R1 et R2 ne peuvent pas représenter un atome d'hydrogène ;
R3 représente un groupe alcényle en (C₂-C₁₀), un groupe benzyle, où le radical benzyle peut être mono- ou poly-substitué par un atome de fluor, un atome de chlore, un atome de brome, un atome d'iode, un groupe OH, un groupe CF₃, un groupe NO₂, un groupe CN, un groupe OCF₃, un groupe O-(C₁-C₆)-alkyle, un groupe alkyle en (C₁-C₆), un groupe NH₂, un groupe NH (C₁-C₆)-alkyle, un groupe N ((C₁-C₆)-alkyle)₂, un groupe SO₂-CH₃, un groupe COOH, un groupe COO-(C₁-C₆)-alkyle, un groupe CONH₂;
R4 et R5 forment conjointement une chaîne alkylène à 3-5 chaînons, dans laquelle un groupe CH₂ est remplacé par un groupe NH, où les groupes alkyle peuvent être mono- ou polysubstitués par un atome de fluor, un atome de chlore, un atome de brome ou un atome d'iode ;
R11 représente un atome d'hydrogène, un groupe alkyle en (C₁-C₆), un groupe cycloalkyle en (C₃-C₈), un groupe (C₁-C₄) -alkylène-aryle ou un groupe (C₁-C₄)-alkylène-hétérocyclyle ;
n vaut 0 ;
ainsi que leurs sels physiologiquement acceptables.

5. Composés selon l'une ou plusieurs des revendications 1 à 4 pour une utilisation en tant que médicament.

6. Médicament comprenant l'un ou plusieurs des composés selon l'une ou plusieurs des revendications 1 à 4.

7. Médicament comprenant l'un ou plusieurs des composés selon l'une ou plusieurs des revendications 1 à 4 et au moins un ingrédient actif supplémentaire.

8. Médicament selon la revendication 7, **caractérisé en ce que** qu'il comprend, en tant qu'ingrédient actif supplémentaire, un ou plusieurs agents antidiabétiques, ingrédients actifs hypoglycémiants, inhibiteurs de réductase HMG-CoA, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption d'acide biliaire, inhibiteurs de CETP, agents adsorbants polymères d'acide biliaire, inducteurs de récepteur de LDL, inhibiteurs d'ACAT, agents antioxydants, inhibiteurs de lipoprotéine-lipase, inhibiteurs d'ATP-citrate-lyase, inhibiteurs de squalène-synthétase, antagonistes de lipoprotéine (a), inhibiteurs de lipase, insulines, sulfonylurées, biguanides, méglitinides, thiazolidinediones, inhibiteurs de α-glucosidase, ingrédients actifs agissant sur le canal potassique ATP-dépendant des cellules bêta, agonistes de CART, agonistes de NPY, agonistes de MC4, antagonistes de l'orexine, agonistes de H3, agonistes de TNF, agonistes de CRF, antagonistes de CRF BP, agonistes de l'urocortine, agonistes de β3, antagonistes de récepteur CB1, agonistes de la MSH (hormone mélano-stimulante), agonistes de CCK, inhibiteurs du recaptage de la sérotonine, composés mixtes sérotoninergiques et noradrénergiques, agonistes de 5HT, agonistes de la bombésine, antagonistes de la galanine, hormones de croissance, composés libérant une hormone de croissance, agonistes de TRH, modulateurs de la protéine découplante 2 ou 3, agonistes de la leptine, agonistes de DA (bromocriptine, doprexine), inhibiteurs de lipase/amylase, modulateurs de PPAR, modulateurs de RXR ou agonistes de TR-β ou amphétamines.

9. Utilisation des composés selon l'une ou plusieurs des revendications 1 à 4 pour la production d'un médicament destiné à réduire la glycémie.

10. Utilisation des composés selon l'une ou plusieurs des revendications 1 à 4 pour la production d'un médicament destiné au traitement du diabète du type II.

11. Utilisation des composés selon l'une ou plusieurs des revendications 1 à 4 pour la production d'un médicament destiné au traitement de troubles du métabolisme lipide et glucidique.

12. Utilisation des composés selon l'une ou plusieurs des revendications 1 à 4 pour la production d'un médicament destiné au traitement de manifestations artério-sclérotiques.

13. Utilisation des composés selon l'une ou plusieurs des revendications 1 à 4 pour la production d'un médicament destiné au traitement de la résistance à l'insuline.

14. Procédé de production d'un médicament comprenant l'un ou plusieurs des composés selon l'une ou plusieurs des revendications 1 à 4, **caractérisé en ce que** l'ingrédient actif est mélangé avec un support pharmaceutiquement approprié et ce mélange est mis sous une forme appropriée pour l'administration.

15. Utilisation des composés of formule I 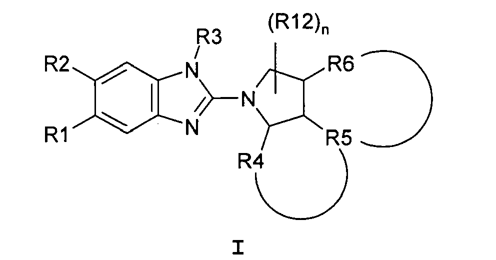 dans laquelle:
R1 et R2 représentent, indépendamment l'un de l'autre, un atome d'hydrogène, un groupe CONR20R21, un groupe NR22COR23 ou un groupe NR24R25 ;
R20 et R21 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle ou un groupe (C₁-C₆) -alkylène- (C₆-C₁₀) -hétéro-cyclyle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O-(C₁-C₆)-alkyle, un groupe S-(C₁-C₆)-alkyle, un groupe S (O) - (C₁-C₆) -alkyle ou un groupe S (O)₂- (C₁-C₆) -alkyle ;
R22 et R23 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆)-alkylène (C₆-C₁₀)-aryle, un groupe (C₁-C₆) -alkylène- (C₆-C₁₀)-hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un -groupe CN, un groupe NO₂, un groupe SH, un groupe OH, un groupe alkyle en (C₁-C₆), un groupe O-(C₁-C₆)-alkyle, un groupe S-(C₁-C₆)-alkyle, un groupe S (O) - (C₁-C₆) -alkyle ou un groupe S (O) ₂- (C₁-C₆) -alkyle ;
R24 et R25 représentent, indépendamment l'un de l'autre, un atome d'hydrogène, un groupe alkyle en (C₁-C₁₀), un groupe cycloalkyle en (C₃-C₁₀), un groupe alcényle en (C₂-C₁₀), un groupe alcynyle en (C₂-C₁₀), un groupe aryle en (C₆-C₁₀), un groupe hétérocyclyle, un groupe (C₁-C₆) -alkylène- (C₆-C₁₀) -aryle, un groupe (C₁-C₆) -alkylène- (C₆-C₁₀) -hétéro-cyclyle ou un groupe S(O)₂-aryle, où les radicaux alkyle, cycloalkyle, alcényle, alcynyle, alkylène, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe O- (C₁-C₆) -alkyle, un groupe S-(C₁-C₆)-alkyle, un groupe S (O) - (C₁-C₆) -alkyle ou un groupe S (O) ₂- (C₁-C₆) -alkyle ;
R3 représente indépendamment un atome d'hydrogène, un groupe alkyle en (C₁-C₁₀), un groupe cycloalkyle en (C₃-C₁₀) un groupe alcényle en (C₂-C₁₀), un groupe alcynyle en (C₂-C₁₀), un groupe aryle en (C₆-C₁₀) ou un groupe hétérocyclyle, où les radicaux alkyle, cycloalkyle, alcényle, alcynyle, aryle et hétérocyclyle peuvent être mono- ou poly-substitués par un atome de fluor, un atome de chlore, un atome de brome, un atome d'iode, un groupe CN, un groupe NO₂, un groupe SH, un groupe OH, un groupe alkyle en (C₁-C₆), un groupe -CF₃, -OCF₃, -SCF₃, un groupe alcényle en (C₂-C₆), un groupe alcynyle en (C₂-C₆), un groupe OR7, un groupe OP(O) (OR7)₂, un groupe NR7R8, un groupe NR7CONR7R8, un groupe COR7, un groupe OCOR7, un groupe OCOOR7, un groupe COOR7, un groupe CONR7R8, un groupe OCONR7R8, un groupe (C₁-C₆) -alkylène-OR7, un groupe (C₁-C₆) -alkylène-NR7R8, un groupe (C₁-C₆) -alkylène-NR7S (O) ₂R7, un groupe (C₁-C₆) -alkylène-SR7, un groupe (C₁-C₆)-alkylène-S (O) R7, un groupe (C₁-C₆)-alkylène-S (O) ₂R7, un groupe (C₁-C₆)-alkylène-S (O) ₂NR7R8, un groupe (C₁-C₆) -alkylène-COR7, un groupe (C₁-C₆) -alkylène-COOR7, un groupe (C₁-C₆)-alkylène-CONR7R8, un groupe SR7, un groupe S(O)R7, un groupe S(O)₂R7, un groupe S(O)₂NR7R8, un groupe NR7S(O)₂R7, un groupe (C₁-C₆) -alkylène- (C₃-Cio) -cycloalkyle, un groupe (C₁-C₆) -alkylène- (C₆-C₁₀) -aryle, un groupe (C₁-C₆)-alkylène-hétérocyclyle, un groupe cycloalkyle en (C₃-C₁₀), un groupe aryle en (C₆-C₁₀) ou un groupe hétérocyclyle ;
R7 et R8 représentent, indépendamment l'un de l'autre, un atome d'hydrogène, un groupe alkyle en (C₁-C₆), un groupe -CF₃, un groupe cycloalkyle en (C₃-C₁₀), un groupe aryle en (C₆-C₁₀), un groupe hétérocyclyle, un groupe (C₁-C₆) -alkylène-CONR9R10, un groupe CONR9R10, un groupe (C₁-C₆)-alkylène-COOR9, un groupe COOR9, un groupe COR9, un groupe (C₁-C₆) -alkylène-COR9, un groupe (C₁-C₆)-alkylène-OR9, un groupe (C₁-C₆)-alkylène-NR9R10, un groupe (C₁-C₆)-alkylène-SR9, un groupe (C₁-C₆) -alkylène-S (O) R9, un groupe (C₁-C₆) -alkylène-S (O)₂R9, un groupe S(O)R9, un groupe S(O)₂R9, un groupe (C₁-C₄)-alkylène- (C₆-C₁₀) -aryle ou un groupe (C₁-C₄)-alkylène-hétérocyclyle ;
R9 et R10 représentent, indépendamment l'un de l'autre, un atome d'hydrogène, un groupe alkyle en (C₁- C₆) un groupe (C₁-C₆)-alkylène- (C₆-C₁₀) -aryle, un groupe aryle en (C₆-C₁₀), un groupe hétérocyclyle ou un groupe (C₁-C₆)-alkylène-hétérocyclyle ;
R4 et R5 forment conjointement une chaîne alkylène à 3-5 chaînons, dans laquelle un groupe CH₂ est remplacé par un groupe NR11, où R6 représente un atome d'hydrogène ou un groupe R12, ou
R5 et R6 forment conjointement une chaîne alkylène à 3-5 chaînons, dans laquelle un groupe CH₂ est remplacé par un groupe NR11, où R4 représente un atome d'hydrogène ou un groupe R12 ; la chaîne alkylène à 3-5 chaînons pouvant être, dans chaque cas, mono- ou poly-substituée par un atome de fluor, un atome de chlore, un atome de brome, un atome d'iode, un groupe alkyle en (C₁-C₆), un groupe cycloalkyle en (C₃-C₈), un groupe NH₂, un groupe NH (C₁-C₆) -alkyle, un groupe NH (C₃-C₇) -cycloalkyle, un groupe N((C₁-C₆)-alkyle)₂ ou un groupe O-(C₁-C₆)-alkyle, où les groupes alkyle peuvent être mono- ou poly-substitués par un atome de fluor, un atome de chlore, un atome de brome ou un atome d'iode ;
R11 représente un atome d'hydrogène, un groupe alkyle en (C₁-C₆), un groupe cycloalkyle en (C₃-C₈), un groupe (C₁-C₄) -alkylène-aryle ou un groupe (C₁-C₄)-alkylène-hétérocyclyle ;
R12 représente un atome de fluor, un atome de chlore, un atome de brome, un atome d'iode, un groupe alkyle en (C₁-C₆), un groupe cycloalkyle en (C₃-C₈), un groupe NH₂, un groupe NH(C₁-C₆) -alkyle, un groupe NH(C₃-C₇)-cycloalkyle, un groupe N ((C₁-C₆) -alkyle) ₂ ou un groupe O- (C₁-C₆) -alkyle, où les groupes alkyle peuvent être mono- ou poly-substitués par un atome de fluor, un atome de chlore, un atome de brome ou un atome d'iode ;
n vaut 0, 1 ou 2 ;
ainsi que leurs sels physiologiquement acceptables pour la production d'un médicament destiné à réduire la glycémie.
